# EUROPÄISCHE PATENTSCHRIFT

(11) **EP 3 595 526 B1**
(45) Veröffentlichungstag und Bekanntmachung des Hinweises auf die Patenterteilung: **07.01.2026**
(21) Anmeldenummer: 18709340.6
(22) Anmeldetag: 05.03.2018
(51) Int. Cl.: A61B 5/145, A61B 5/1477, A61B 5/00, G16H 40/63, G16H 50/20

(54) **VERFAHREN ZUM ERMITTELN EINES HAUTZUSTANDS**
METHOD FOR ASCERTAINING A SKIN CONDITION
PROCÉDÉ POUR DÉTERMINER UN ÉTAT CUTANÉ

(30) Priorität: 17.03.2017 DE 102017204493
(43) Veröffentlichungstag der Anmeldung: 22.01.2020
(73) Patentinhaber: Henkel AG & Co. KGaA, 40589 Düsseldorf (DE)
(72) Erfinder: BOCK, Andreas, 41464 Neuss (DE); WELSS, Thomas, 40591 Düsseldorf (DE); HUNDEIKER, Claudia, 40667 Meerbusch (DE); WALDMANN-LAUE, Marianne, 40789 Monheim (DE)
(86) Internationale Anmeldenummer: PCT/EP2018/055354
(87) Internationale Veröffentlichungsnummer: WO 2018/166830

(56) Entgegenhaltungen:
- WO-A1-2016/090189
- WO-A1-2017/037352
- WO-A2-2016/007944
- WO-A2-2016/156585
- US-A1- 2017 061 822

## Beschreibung

Verschiedene Ausführungsformen betreffen im Allgemeinen ein Verfahren und eine Vorrichtung zum Ermitteln eines Hautzustands, ein Verfahren und eine Vorrichtung zum Ermitteln eines Gehalts von Spurenelementen in der Haut und ein Verfahren zum Ermitteln einer kosmetischen Hautbehandlungsempfehlung.

Hierin sind unter Spurenelementen für den Menschen essentielle chemische Elemente zu verstehen, welche in Massenanteilen von weniger als 50 mg/kg im Organismus vorkommen, und wegen seiner Wirkungsweise trotz seines höheren Massenanteils außerdem Eisen.

Im Laufe des Alterns kann Haut Spurenelemente verlieren, welche für die Hautphysiologie wichtig sein können. Beispielsweise können wichtige Enzyme der Haut diese für oder als so genannte Cofaktoren benötigen, ohne welche die Enzyme nicht aktiv werden können.

Für eine Pflege speziell von spurenelementarmer Haut können verschiedene spezielle Kosmetikprodukte für das Gesicht und/oder den Körper erhältlich sein. Allerdings kann eine Wirksamkeit der kosmetischen Produkte stark von einer individuellen Physiologie der Haut abhängen.

Ohne fachkundige dermatologische oder kosmetische Beratung kann es für einen Nutzer schwer sein, seinen individuellen Hautzustand und die für seinen Hautzustand geeigneten Kosmetika zu ermitteln. Ein Spurenelementgehalt von Blut des Nutzers kann außerdem ungeeignet sein, um einen Haut-Spurenelementgehalt zu ermitteln, da beispielsweise die Epidermis als äußerste Barriereschicht der Haut nicht mit dem Blutkreislauf verbunden ist.

Sofern der Nutzer dennoch kosmetische Produkte konsumiert, kann es praktisch unmöglich sein, einen Behandlungserfolg nachzuvollziehen, weil es dem Nutzer, z.B. zu Hause, an Möglichkeiten fehlt, standardisiert und objektiv ein Behandlungsergebnis zu beurteilen.

Dadurch kann es dem Konsumenten erschwert sein, eine individuelle Wirksamkeit eines Kosmetikums zu beurteilen, was dazu führen kann, dass eine Motivation, eine entsprechende kosmetische Behandlung, beispielsweise längerfristig, durchzuführen, beeinträchtigt sein kann. Dies kann selbst dann der Fall sein, wenn ein Kosmetikprodukt geeignet wäre, eine objektiv nachweisbare erwünschte Wirkung zu erzielen.

In vielen Bereichen des täglichen Lebens gibt es seit einiger Zeit einen Trend zu personalisierten Programmen, die auf individuelle Voraussetzungen und Bedürfnisse gezielt eingehen können, beispielsweise in einem Ernährungs- oder Gesundheitsbereich, aber auch in einem Bereich personalisierter Kosmetik. Diese kann es einem Nutzer ermöglichen, gezielt Kosmetikprodukte zu finden und/oder Pflegehinweise zu erhalten, die auf individuelle Bedürfnisse seiner Haut abgestimmt sind, und somit eine besonders hohe Wirksamkeit ermöglichen.

Dokument WO2016007944 beschreibt eine Vorrichtung zur Messung des Schweißes eines Nutzers. Dokument WO2017037352 beschreibt eine Gesichtsmaske, die Sensorelemente zur Bestimmung des Hautzustands eines Nutzers und Mittel zum Auftragen eines kosmetischen Produkts über die Gesichtsmaske auf das Gesicht des Nutzers umfasst.

Die Erfindung ist im beigefügten Anspruchssatz beschrieben.

In verschiedenen Ausführungsbeispielen kann eine nicht beanspruchte Vorrichtung zum Ermitteln eines Hautzustands eines Nutzers unter Einbeziehung eines ermittelten Spurenelementgehalts bereitgestellt werden. Das Ermitteln des Gehalts an Spurenelementen auf bzw. in der Haut und ein kontinuierliches Überprüfen von Veränderungen können in verschiedenen Ausführungsbeispielen dem Nutzer einen Zusatznutzen einer individualisierbaren Behandlung bieten.

In verschiedenen Ausführungsbeispielen kann zum Ermitteln des Hautzustands bzw. des Spurenelementgehalts der Haut ein Sensor verwendet werden, welcher einen Gehalt an essentiellen Spurenelementen ausgewählt aus der Gruppe bestehend aus Chrom, Cobalt, Eisen, Kupfer, Mangan, Molybdän, Selen, Silicium, Zink, Zinn, Vanadium, Nickel, Arsen, Barium, Strontium, Aluminium und Kombinationen davon in der Haut detektiert (genauer kann ein Sensor-Messwert erfasst und daraus der Spurenelementgehalt für eines der Spurenelemente ermittelt werden). Dafür kann eine spezielle Vorrichtung genutzt werden, welche an ein Smartphone, ein Tablet, Smart Mirror oder eine andere Datenverarbeitungsvorrichtung (z.B. einen Computer) anschließbar oder darin integriert sein kann.

In verschiedenen Ausführungsbeispielen kann mittels einer App oder einer sonstigen Software der detektierte Spurenelementgehalt ermittelt und in Form eines Wertes (z.B. mit willkürlichen Einheiten), als graphische Darstellung und/oder akustische Mitteilung bereitgestellt werden.

In verschiedenen Ausführungsbeispielen kann anhand des Spurenelementgehalts ein Status hinsichtlich des Gehalts von Spurenelementen über einen längeren Zeitraum aufgezeichnet werden. In verschiedenen Ausführungsbeispielen kann anhand des Spurenelementgehalts eine Folge einer Dysbalance beschrieben werden.

In verschiedenen Ausführungsbeispielen kann anhand des Spurenelementgehalts eine individualisierte kosmetische Behandlung zur Optimierung des Zustands empfohlen werden.

In verschiedenen Ausführungsbeispielen können als Sensor selektive Chemosensoren genutzt werden. In verschiedenen Ausführungsbeispielen können als Sensor ionenselektive Elektroden (ISE) genutzt werden, z.B. chipbasierte Chalkogenidglas-ISE. In verschiedenen Ausführungsbeispielen können andere geeignete Sensoren und Techniken zur Quantifizierung von Spurenelementen der Haut verwendet werden.

Die Vorrichtung kann in verschiedenen Ausführungsbeispielen günstig und klein (z.B. tragbar) ausgebildet sein. Die Vorrichtung kann an einem (lebenden) Nutzer angewendet werden, beispielsweise bei dem Nutzer zu Hause, an einem Verkaufspunkt für Kosmetikladen, bei einem Hautarzt und/oder in einem Kosmetikstudio. Die Vorrichtung kann in verschiedenen Ausführungsbeispielen ein Smartphone, ein Tablet, ein iPad oder eine ähnliche elektronische Schaltkreisvorrichtung (z.B. eine Datenverarbeitungsvorrichtung und/oder eine Datenübertragungsvorrichtung) aufweisen, und eine integrierte Vorrichtung oder eine Zusatzvorrichtung, die beispielsweise an der elektronischen Schaltkreisvorrichtung anbringbar und/oder damit verbindbar (z.B. mittels einer Datenverbindung verbindbar) sein kann. Die integrierte Vorrichtung bzw. die Zusatzvorrichtung (ggf. sogar die gesamte Vorrichtung) kann eine Größe aufweisen, die es ermöglicht, sie problemlos in einer Hand- oder Hosentasche unterzubringen, beispielsweise mit einer Fläche von weniger als 36 cm² und mit einer Dicke von weniger als 2 cm.

In verschiedenen Ausführungsbeispielen kann mittels der Vorrichtung zum Ermitteln eines Hautzustands der Spurenelementgehalt des Nutzers unter Verwendung von Elektroden bestimmt werden. Für das Bestimmten des Spurenelementgehalts des Nutzers kann in verschiedenen Ausführungsbeispielen eine Mehrzahl von Elektroden in Kontakt mit Haut des Nutzers gebracht werden, wobei eine der Elektroden (auch als erste Elektrode bezeichnet) die Haut indirekt mittels einer Selektionsschicht kontaktieren kann. In verschiedenen Ausführungsbeispielen kann die mindestens eine weitere Elektrode (auch als zweite Elektrode bezeichnet) die Haut direkt berühren. In verschiedenen Ausführungsbeispielen kann zwischen der Mehrzahl von Elektroden und der Haut ein Lösungsmittel (z.B. Wasser) angeordnet sein, z.B. als Lösungsmittelschicht. Die in der Haut vorhandenen Spurenelemente können in verschiedenen Ausführungsbeispielen zumindest teilweise in das Lösungsmittel übertreten und dort mittels der Mehrzahl von Elektroden erfassbar sein (beispielsweise jeweils eines der Spurenelemente pro Mehrzahl, z.B. Paar oder Triplett, von Elektroden).

In verschiedenen Ausführungsbeispielen kann die Selektionsschicht mindestens einen reaktiven Bestandteil aufweisen, der eingerichtet sein kann, unter Freisetzung von Elektronen oder Ionen mit dem Spurenelement zu reagieren, wobei die freigesetzten Elektronen oder Ionen eingerichtet sein können, die erste Elektrode zu erreichen, um ein von einer Konzentration des Spurenelements abhängiges Signal zu erzeugen.

In verschiedenen Ausführungsbeispielen kann die Selektionsschicht eine teildurchlässige Barriereschicht aufweisen, welche eingerichtet sein kann, nur für das Spurenelement durchlässig zu sein, derart, dass nur für das Spurenelement die erste Elektrode nach einem Hindurchtreten durch die Barriereschicht erreichbar ist, um das von der Konzentration des Spurenelements abhängige Signal zu erzeugen.

In verschiedenen Ausführungsbeispielen kann das unter Verwendung der Elektroden ermittelte Signal verglichen werden mit Referenzsignalen für bekannte Spurenelementgehalte und anhand dessen ein Spurenelementgehalt des Nutzers ermittelt werden (wobei das Vergleichen auch derart erfolgen kann, dass das Signal mittels einer anhand von Referenzsignalen und Referenzdaten ermittelten Beziehung zu einem Spurenelementgehalt des Nutzers umgerechnet wird). Die Referenzdaten können in verschiedenen Ausführungsbeispielen vorab ermittelt worden sein, beispielsweise bei Laborversuchen.

In verschiedenen Ausführungsbeispielen kann das Vergleichen des Signals mit dem Referenzsignal und/oder das Ermitteln des Spurenelementgehalts daraus mittels der elektronischen Schaltkreisvorrichtung (z.B. direkt) erfolgen, beispielsweise mittels einer App oder eines sonstigen Softwareprogramms, welches von der elektronischen Schaltkreisvorrichtung betrieben wird. Die elektronische Schaltkreisvorrichtung kann beispielsweise ein Smartphone, ein Tablet, ein iPad oder ähnliches sein.

In verschiedenen Ausführungsbeispielen kann das Vergleichen des Signals mit dem Referenzsignal und/oder das Ermitteln des Spurenelementgehalts daraus indirekt mittels der elektronischen Schaltkreisvorrichtung erfolgen, beispielsweise indem die elektronische Schaltkreisvorrichtung eine (z.B. kontaktlose) Datenkommunikationsverbindung bereitstellt und mittels der Datenkommunikationsverbindung das Signal und/oder das Ergebnis des Vergleichs des Signals mit dem Referenzsignal einer externen Datenverarbeitungsvorrichtung (z.B. einer Cloud) bereitstellt und von der externen Datenverarbeitungsvorrichtung den mittels des Signals und/oder des Ergebnisses des Vergleichs ermittelten Spurenelementgehalt empfängt.

Dem Nutzer kann der ermittelte Spurenelementgehalt in verschiedenen Ausführungsbeispielen auf beliebige, z.B. vom Nutzer wählbare, Art bereitgestellt werden, z.B. als ein Zahlenwert (eine Hautspurenelementgehaltmaßzahl), eine graphische Darstellung (beispielsweise einer Darstellung des ermittelten Werts in im Verhältnis zu einem gesamten Wertebereich von sehr spurenelementarm über normal bis hin zu sehr spurenelementreich), eine akustische Mitteilung, oder ähnliches.

In verschiedenen Ausführungsbeispielen kann die Vorrichtung ferner eingerichtet sein, basierend auf dem ermittelten Spurenelementgehalt einen Hautzustand zu ermitteln. In einem Fall, in dem für nur einen Hautbereich der Spurenelementgehalt ermittelt wird bzw. wurde, kann der Hautzustand, der basierend darauf ermittelt wird, als einheitlicher Hautzustand für den einen ermittelten Spurenelementgehalt ermittelt werden, z.B. sehr spurenelementarme Haut bei sehr geringem Spurenelementgehalt, sehr spurenelementreiche Haut bei sehr hohem Spurenelementgehalt, normale Haut bei normalem Spurenelementgehalt, usw., mit einer beliebigen Anzahl von Abstufungen (z.B. dazwischen). In einem Fall, in dem für eine Mehrzahl von Hautbereichen der Spurenelementgehalt ermittelt wird bzw. wurde, kann der Hautzustand, der basierend darauf ermittelt wird, in verschiedenen Ausführungsbeispielen als ein einziger Hautzustand für die Mehrzahl von ermittelten Spurenelementgehalten ermittelt werden, z.B. als Mischhaut mit Bereichen spurenelementarmer Haut und Bereichen spurenelementreicher Haut (z.B. bei für mindestens einen ersten Hautbereich ermitteltem geringem Spurenelementgehalt und für mindestens einen zweiten Hautbereich ermitteltem hohem Spurenelementgehalt).

In verschiedenen Ausführungsbeispielen kann der Hautzustand numerisch angegeben werden, beispielsweise als ein Summenwert, wodurch eine Plakativität (z.B. der Darstellung des Ergebnisses) erhöht werden kann.

Insbesondere in einem Fall, in welchem der Spurenelementgehalt für eine Mehrzahl von Hautbereichen ermittelt wird, kann das Bereitstellen des Hautzustands an den Nutzer eine graphische Darstellung aufweisen, beispielsweise eine Anzeige, z.B. mittels einer Anzeigevorrichtung. In der graphischen Darstellung kann der ermittelte Spurenelementgehalt der Mehrzahl von Bereichen mit einer Codierung anhand des Spurenelementgehalts in einer Darstellung des Nutzers (z.B. einer schematischen Darstellung oder auf einem Foto des Nutzers) angezeigt werden. Beispielsweise können in einer schematischen Darstellung eines Gesichts des Nutzers Bereiche unterschiedlichen Spurenelementgehalts mit unterschiedlichen Farben und/oder Mustern dargestellt werden, z.B. Bereiche mit normalem Spurenelementgehalt grün, mit niedrigem Spurenelementgehalt rot und mit hohem Spurenelementgehalt blau oder ähnliches. In einem anderen Beispiel können einem Foto des Nutzers, z.B. einem digitalen Foto, welches das Gesicht des Nutzers zeigt, unterschiedliche Muster überlagert sein, z.B. ein Punktmuster für Bereiche mit hohem Spurenelementgehalt und ein Linienmuster für Bereiche mit niedrigem Spurenelementgehalt, wobei Bereiche normaler Haut ungemustert verbleiben können, oder ähnliches.

Beim graphischen Darstellen des Hautzustands, z.B. des Spurenelementgehalts des mindestens einen Hautbereichs, z.B. der Mehrzahl der Hautbereiche, kann in verschiedenen Ausführungsbeispielen nur für den Bereich/die Bereiche, für den/die der Spurenelementgehalt ermittelt wurde, der Spurenelementgehalt dargestellt werden. In verschiedenen Ausführungsbeispielen kann ein Bereich, für den ein ermittelter Spurenelementgehalt angezeigt wird, über den Hautbereich hinaus, für den der Spurenelementgehalt ermittelt wurde, extrapoliert werden, beispielsweise unter Einbeziehung typischer Hautspurenelementerteilungsmuster.

Dabei kann in verschiedenen Ausführungsbeispielen eine Information darüber, wo der Bereich/die Bereiche ist/sind, der elektronischen Schaltkreisvorrichtung bereitgestellt werden, beispielsweise mittels des Nutzers, z.B. mittels einer Eingabevorrichtung (z.B. Antippen eines berührungsempfindlichen Displays, auf welchem eine schematische oder fotografische Darstellung des Nutzers angezeigt wird, mittels Texteingabe über eine Tastatur oder jede andere geeignete Eingabemöglichkeit).

In verschiedenen Ausführungsbeispielen kann die Anzeigevorrichtung Teil der Vorrichtung sein, z.B. ein Display eines Smartphones, Tablets oder iPads. In verschiedenen Ausführungsbeispielen kann die Anzeigevorrichtung mit der Vorrichtung gekoppelt sein, beispielsweise mittels einer (z.B. kabellosen) Datenverbindung.

Zusätzlich oder alternativ kann in verschiedenen Ausführungsbeispielen dem ermittelten Hautzustand ein Qualitätswert zugeordnet werden, mit einem hohen Qualitätswert für normale/gesunde Haut und einem niedrigen Qualitätswert für zu spurenelementarme/zu spurenelementreiche Haut.

In verschiedenen Ausführungsbeispielen kann die elektronische Schaltkreisvorrichtung eingerichtet sein, selbst, d.h. direkt, den Hautzustand zu ermitteln, beispielsweise mittels einer Software, z.B. einer App, die auf der elektronischen Schaltkreisvorrichtung installiert sein kann.

In verschiedenen Ausführungsbeispielen kann die elektronische Schaltkreisvorrichtung eingerichtet sein, indirekt den Hautzustand zu ermitteln, beispielsweise indem die elektronische Schaltkreisvorrichtung das Ermitteln des Hautzustands mittels der externen Datenverarbeitungsvorrichtung, z.B. mittels einer Software, z.B. einer App, die auf der externen Datenverarbeitungsvorrichtung installiert sein kann, veranlasst, beispielsweise ähnlich wie oben für das Ermitteln des Spurenelementgehalts beschrieben.

In verschiedenen Ausführungsbeispielen kann die Software/App zum Ermitteln des Spurenelementgehalts dieselbe sein wie die Software/App zum Ermitteln des Hautzustands. In verschiedenen Ausführungsbeispielen, z.B. in einem Fall, dass der Spurenelementgehalt mittels der externen Datenverarbeitungsvorrichtung ermittelt wird, wohingegen der Hautzustand von der elektronische Schaltkreisvorrichtung ermittelt wird, oder umgekehrt, kann/können unterschiedliche Software/Apps für das Ermitteln des Spurenelementgehalts und für das Ermitteln des Hautzustands verwendet werden.

In verschiedenen Ausführungsbeispielen können ein Verfahren und eine Vorrichtung zum Ermitteln eines Hautzustands bereitgestellt werden. Dafür kann in verschiedenen Ausführungsbeispielen ein Spurenelementgehalt ermittelt werden. Die Vorrichtung, das Verfahren, und/oder der mittels der Vorrichtung bzw. des Verfahrens ermittelte Hautzustand kann/können genutzt werden, um die Bedürfnisse der Haut des Nutzers zu ermitteln.

In verschiedenen Ausführungsbeispielen soll es einem Nutzer ermöglicht werden, gezielt kosmetische Produkte zu finden und/oder Pflegeratschläge zu erhalten, die auf individuelle Bedürfnisse einer Haut des Nutzers abgestimmt sein können.

Für das Ermitteln des Spurenelementgehalts und/oder für das Ermitteln des Hautzustands kann in verschiedenen Ausführungsbeispielen eine Software, beispielsweise eine App, genutzt werden, welche beispielsweise auf einer tragbaren Datenverarbeitungsvorrichtung (z.B. einem Smartphone, einem Tablet, einem iPad oder ähnlichem) installiert sein kann. Eine Mehrzahl von Elektroden, welche derart gestaltet sein kann, dass nur das Spurenelement, dessen Gehalt in der Haut zu ermitteln ist, geeignet ist, zwischen den Elektroden ein von einer Konzentration des Spurenelements abhängiges messbares Signal zu erzeugen, kann an die Datenverarbeitungsvorrichtung angeschlossen oder darin integriert sein. Mittels der Mehrzahl von Elektroden kann der Spurenelementgehalt der Haut ermittelt werden. Ferner kann, beispielsweise anhand einer Spurenelementgehaltmessung für einen einzelnen Hautbereich oder anhand einer Mehrzahl von Spurenelementgehaltmessungen für unterschiedliche Hautbereiche, ein Hautzustand ermittelt werden, z.B. spurenelementreiche Haut, normale Haut, spurenelementarme Haut oder Mischhaut, welche sowohl Bereiche spurenelementarmer Haut als auch Bereiche spurenelementreicher Haut aufweist.

In verschiedenen Ausführungsbeispielen können mittels der tragbaren Datenverarbeitungsvorrichtung, z.B. mittels der Software/App, der ermittelte Spurenelementgehalt und/oder der ermittelte Hautzustand als Werte (z.B. mit willkürlichen Einheiten), als verbale Mitteilung, als graphische Darstellung (z.B. als schematische bildliche Darstellung, welche Hautbereiche spurenelementreicher/spurenelementarmer/normaler Haut aufweisen), o.ä. dargestellt bzw. mitgeteilt werden.

Es kann bevorzugt sein, dass die elektronische Schaltkreisvorrichtung und die tragbare Datenverarbeitungsvorrichtung identisch miteinander sind.

In alternativen Ausführungsformen der Erfindung können der ermittelte Spurenelementgehalt und/oder der ermittelte Hautzustand mittels eines Smart Mirrors, z.B. mittels der Software/App, als graphische Darstellung (z.B. als schematische bildliche Darstellung, welche Hautbereiche spurenelementreicher/spurenelementarmer/normaler Haut aufweisen), o.ä. auf diesem dargestellt werden.

In verschiedenen Ausführungsbeispielen kann dem Nutzer, basierend auf dem ermittelten Hautzustand, mindestens ein Kosmetikprodukt empfohlen werden. Das Kosmetikprodukt kann geeignet sein, den Hautzustand des Nutzers zu erhalten oder zu verbessern (z.B. einen Spurenelementgehalt zu normalisieren). In verschiedenen Ausführungsbeispielen kann das Kosmetikprodukt eine Creme, eine Lotion, eine Salbe, ein Öl, eine Emulsion, ein Gel, eine Seife, eine Maske, eine Ampulle mit einem Zusatzpflegeprodukt, ein Gesichtswasser, ein Serum, einen Spray oder ähnliches aufweisen. Das Kosmetikprodukt kann neben der Eignung den Hautzustand des Nutzers zu erhalten oder zu verbessern, weitere Eigenschaften, wie beispielsweise pflegende und/oder reinigende Eigenschaften, besitzen.

Zusätzlich können dem Nutzer Nahrungsmittel und/oder Nahrungsergänzungsmittel empfohlen werden, welche geeignet sind, den Spurenelementgehalt zu normalisieren, und ein oder mehrere Spurenelemente enthalten, an dem/denen in der Haut des Nutzers, zumindest in einigen Hautbereichen, ein Mangel vorliegt.

In verschiedenen Ausführungsbeispielen können anhand des ermittelten Spurenelementgehalts und/oder anhand des ermittelten Hautzustands Produktempfehlungen für individuell für den Nutzer passende Kosmetikprodukte und/oder individuelle Pflegehinweise abgeleitet werden. Die Produktempfehlungen und/oder Pflegehinweise können beispielsweise mittels einer Software, z.B. einer App, bereitgestellt werden. Beispielsweise kann dem Nutzer empfohlen werden, ein Kosmetikprodukt, welches mindestens ein Spurenelement aufweist, für welches der ermittelte Spurenelementgehalt der Haut des Nutzers für mindestens einen Hautbereich einen Mangel aufweist, an dem Hautbereich, der den Mangel aufweist, anzuwenden, z.B. eine Spurenelementhaltige Creme dort aufzutragen.

In verschiedenen Ausführungsbeispielen kann die Software/App, welche den Spurenelementgehalt und/oder den Hautzustand ermittelt, dieselbe sein, die die Produktempfehlung und/oder die Pflegehinweise ermittelt. In verschiedenen Ausführungsbeispielen können unterschiedliche Softwareprogramme/Apps verwendet werden für einen Teil der verschiedenen Vorgänge oder alle verschiedenen Vorgänge (Ermitteln des Spurenelementgehalts, Ermitteln des Hautzustands, Ermitteln einer Produktempfehlung, Ermitteln eines Pflegehinweises).

In verschiedenen Ausführungsbeispielen kann ein Behandlungserfolg bei einer kosmetischen Behandlung, welche ein positives Beeinflussen des ermittelten Spurenelementgehalts bzw. des ermittelten Hautzustands zum Ziel haben kann, überwacht werden. In verschiedenen Ausführungsbeispielen kann die Software/App eine Kontrolle und/oder Nachverfolgung der Ergebnisse mittels einer Darstellung (z.B. einer graphischen Darstellung) der Messergebnisse im Verlauf der Zeit ermöglichen.

In verschiedenen Ausführungsbeispielen können beim Ermitteln der Produkt- und Pflegeempfehlungen ferner Informationen hinsichtlich eines generellen Gesundheitszustands, eines Hautzustands, Ernährungsgewohnheiten und weiterer Verhaltensweisen des Nutzers (z.B. tägliche Aufenthaltsdauer im Freien/in der Sonne/im Wasser (z.B. unter anderem in mineralstoffreichen Heilbädern, die auch reich an Spurenelementen sein können), Rauchgewohnheiten, Ernährungsgewohnheiten, usw.) verwendet werden, z.B. mittels der Software/App. Diese Informationen können in verschiedenen Ausführungsbeispielen mittels der Software/App vom Nutzer erfragt werden.

In verschiedenen Ausführungsbeispielen können zum Beurteilen einer Eignung eines Kosmetikprodukts und/oder eines Pflegehinweises zum Pflegen einer Haut mit einem gegebenen Hautzustand Literaturdaten zu Grunde gelegt werden.

In verschiedenen Ausführungsbeispielen kann jedem Hautzustand einer Mehrzahl von Hautzuständen ein Qualitätswert zugeordnet sein oder werden. Ein Kosmetikprodukt und/oder ein Pflegehinweis kann für einen Hautzustand als geeignet bewertet werden, wenn zu erwarten ist, z.B. aufgrund von Literaturdaten, Versuchsergebnissen oder Erfahrungswerten (welche beispielsweise fortlaufend, z.B. auch während einer Nutzung der Software/der App durch den Nutzer, mit neuen Erfahrungswerten anderer Nutzer aktualisiert werden können, siehe unten), dass bei einer (z.B. regelmäßigen) Anwendung des Kosmetikprodukts und/oder des Pflegehinweises der Hautzustand des Nutzers aufrechterhalten wird oder sich zu einem Hautzustand mit einem höheren (d.h. besseren) Qualitätswert ändert.

In verschiedenen Ausführungsbeispielen kann eine Beurteilung einer Eignung eines Kosmetikproduktes, einen Hautzustand zu verbessern, bestätigt oder abgeändert werden mittels eines Aufnehmens von Erfahrungswerten weiterer Nutzer mit demselben oder einem ähnlichen Hautzustand, beispielsweise von Erfahrungswerten hinsichtlich eines Behandlungserfolgs. Vorzugsweise weisen die weiteren Nutzer neben demselben oder ähnlichen Hautzustand auch dasselbe oder ein ähnliches Profil in Bezug auf Alter, Geschlecht und/oder Verhaltensweisen auf. Damit kann ermöglicht sein, dass der Nutzer immer eine optimale Empfehlung erhält.

In verschiedenen Ausführungsbeispielen können eine Kontrolle und eine Nachverfolgung einer Wirksamkeit einer kosmetischen Behandlung auf objektive und standardisierte Art und Weise ermöglicht sein. Die kosmetische Behandlung kann in verschiedenen Ausführungsbeispielen das Ziel haben, einen Spurenelementgehalt zu normalisieren.

In verschiedenen Ausführungsbeispielen kann eine Wirksamkeit einer (z.B. kosmetischen) Behandlung besser nachvollzogen werden und dadurch eine Auswahl eines individuell geeigneten Produkts vereinfacht sein oder werden.

In verschiedenen Ausführungsbeispielen kann eine Motivation eines Nutzers erhöht werden, eine kosmetische Behandlung längerfristig durchzuführen, beispielsweise mittels eines Vergleichs mit anderen Nutzern, z.B. mittels von den anderen Nutzern bereitgestellten Informationen über Behandlungserfolge.

Als Teil des erfindungsgemäßen Verfahrens wird ein Verfahren zum Ermitteln eines Hautzustands bereitgestellt. Das Verfahren weist auf:
ein Ermitteln eines Spurenelementgehalts von Haut, aufweisend: für mindestens einen Hautbereich eines Nutzers ein Anordnen einer Mehrzahl von Elektroden über dem Hautbereich, wobei die Mehrzahl von Elektroden mindestens eine erste Elektrode und eine zweite Elektrode aufweist, wobei die erste Elektrode eine auf dem Hautbereich angeordnete Selektionsschicht kontaktiert und die zweite Elektrode den Hautbereich kontaktiert, wobei die Selektionsschicht derart selektiv für ein Spurenelement gestaltet ist, dass nur das Spurenelement geeignet ist, bei einem Kontakt mit der Selektionsschicht ein von einer Konzentration des Spurenelements abhängiges, zwischen der ersten und der zweiten Elektrode messbares Signal zu erzeugen, ein Messen des von der Konzentration des Spurenelements abhängigen Signals, ein Vergleichen des gemessenen Signals mit Referenzsignalen für bekannte Spurenelementgehalte von Haut, und ein Ermitteln des Hautzustands basierend auf dem ermittelten Spurenelementgehalt der Haut.

In verschiedenen Ausführungsbeispielen kann die Selektionsschicht mindestens einen reaktiven Bestandteil aufweisen, der eingerichtet sein kann, unter Freisetzung von Elektronen oder Ionen mit dem Spurenelement zu reagieren, und wobei die freigesetzten Elektronen oder Ionen eingerichtet sein können, die erste Elektrode zu erreichen, um das von der Konzentration des Spurenelements abhängige Signal zu erzeugen.

In verschiedenen Ausführungsbeispielen kann die Selektionsschicht eine teildurchlässige Barriereschicht aufweisen, welche eingerichtet sein, nur für das Spurenelement durchlässig zu sein, derart, dass nur für das Spurenelement die erste Elektrode nach einem Hindurchtreten durch die Barriereschicht erreichbar sein kann, um das von der Konzentration des Spurenelements abhängige Signal zu erzeugen.

In verschiedenen Ausführungsbeispielen kann das Verfahren vor dem Anordnen der Mehrzahl von Elektroden über dem mindestens einen Hautbereich ferner ein Benetzen des mindestens einen Hautbereichs mittels eines Lösungsmittels aufweisen.

In verschiedenen Ausführungsbeispielen kann das Lösungsmittel Wasser aufweisen.

In verschiedenen Ausführungsbeispielen kann das Spurenelement eines aufweisen aus einer Gruppe von Spurenelementen, wobei die Gruppe Chrom, Cobalt, Eisen, Kupfer, Mangan, Molybdän, Selen, Silicium, Zink, Zinn, Vanadium, Nickel, Arsen, Barium, Strontium und Aluminium aufweisen kann. Vorzugsweise ist das Spurenelement ausgewählt aus der Gruppe bestehend aus Chrom,

Cobalt, Eisen, Kupfer, Mangan, Molybdän, Selen, Silicium, Zink und Kombinationen.

In verschiedenen Ausführungsbeispielen kann der mindestens eine Hautbereich eine Mehrzahl von Hautbereichen des Nutzers aufweisen.

In verschiedenen Ausführungsbeispielen kann die Mehrzahl von Elektroden Teil einer tragbaren Vorrichtung sein.

In verschiedenen Ausführungsbeispielen kann die tragbare Vorrichtung ein Smartphone, ein Tablet oder ein iPad aufweisen oder an einem Smartphone, einem Tablet oder einem iPad anbringbar sein.

In verschiedenen Ausführungsbeispielen kann das Vergleichen des gemessenen Signals mit Referenzsignalen für bekannte Spurenelementgehalte von Haut und das Ermitteln des Hautzustands basierend auf dem ermittelten Spurenelementgehalt der Haut mittels einer App erfolgen.

Erfindungsgemäß wird ein Verfahren zum Ermitteln einer kosmetischen Hautbehandlungsempfehlung bereitgestellt. Das Verfahren weist ein Ermitteln eines Hautzustands gemäß dem oben beschriebenen Teilverfahren und ein Ermitteln des Kosmetikprodukts und/oder einer Pflegeanweisung basierend auf dem ermittelten Hautzustand und einer Datenbank auf, wobei die Datenbank eine Mehrzahl von Hautzuständen und eine Mehrzahl von zugeordneten Kosmetikprodukten und/oder Pflegeanweisungen aufweist wobei jedem Hautzustand der Mehrzahl von Hautzuständen mindestens ein geeignetes Kosmetikprodukt und/oder mindestens eine Pflegeanweisung zugeordnet sind.

In verschiedenen Ausführungsbeispielen kann jedem Hautzustand der Mehrzahl von Hautzuständen ein Qualitätswert zugeordnet sein, und das Kosmetikprodukt und/oder die Pflegeanweisung kann geeignet sein für den Hautzustand der Mehrzahl von Hautzuständen, wenn basierend auf gespeicherten Erfahrungswerten mit dem Kosmetikprodukt eine Verbesserung oder Aufrechterhaltung des Qualitätswerts des Hautzustands zu erwarten ist.

In verschiedenen Ausführungsbeispielen kann das Verfahren ferner ein Aktualisieren der Datenbank basierend auf neuen Erfahrungswerten von einer Mehrzahl von Nutzern aufweisen.

Erfindungsgemäß ist der ermittelte Hautzustand ein Mangelzustand hinsichtlich des Spurenelements, und das Kosmetikprodukt weist das Spurenelement auf.

In verschiedenen Ausführungsbeispielen kann das Ermitteln des Gehalts eines Spurenelements eines Hautbereichs und/oder das Ermitteln des Hautzustands ein Übertragen des Signals, eines Ergebnisses des Vergleichs und/oder des ermittelten Gehalts des Spurenelements des Hautbereichs an eine externe Datenverarbeitungsvorrichtung und ein Empfangen des Gehalts des Spurenelements des Hautbereichs und/oder des Hautzustands aufweisen.

In verschiedenen Ausführungsbeispielen wird eine nicht beanspruchte Vorrichtung zum Ermitteln eines Hautzustands bereitgestellt. Die Vorrichtung kann aufweisen: eine Mehrzahl von Elektroden, wobei die Mehrzahl von Elektroden mindestens eine erste Elektrode und eine zweite Elektrode aufweist, eine Selektionsschicht, wobei die Selektionsschicht derart selektiv für das Spurenelement gestaltet ist, dass nur das Spurenelement geeignet ist, bei einem Kontakt mit der Selektionsschicht ein von einer Konzentration des Spurenelements abhängiges, zwischen der ersten und der zweiten Elektrode messbares Signal zu erzeugen, wobei die erste Elektrode, die zweite Elektrode und die Selektionsschicht derart gestaltet sind, dass die Mehrzahl von Elektroden und die Selektionsschicht derart über einem Hautbereich eines Nutzers anordenbar sind, dass die Selektionsschicht den Hautbereich kontaktiert, die erste Elektrode die Selektionsschicht kontaktiert, und die zweite Elektrode den Hautbereich kontaktiert, und eine elektronische Schaltkreisvorrichtung, welche eingerichtet ist zum Messen des von der Konzentration des Spurenelements abhängigen Signals, zum Ermitteln des Spurenelementgehalts anhand eines Vergleichs zwischen dem gemessenen Signal und Referenzsignalen für bekannte Spurenelementgehalte von Haut und zum Ermitteln des Hautzustands anhand des Spurenelementgehalts.

In verschiedenen Ausführungsbeispielen kann die Selektionsschicht mindestens einen reaktiven Bestandteil aufweisen, der eingerichtet ist, unter Freisetzung von Elektronen oder Ionen mit dem Spurenelement zu reagieren, wobei die freigesetzten Elektronen oder Ionen eingerichtet sein können, die erste Elektrode zu erreichen, um das von der Konzentration des Spurenelements abhängige Signal zu erzeugen.

In verschiedenen Ausführungsbeispielen kann die Selektionsschicht eine teildurchlässige Barriereschicht aufweisen, welche eingerichtet sein kann, nur für das Spurenelement durchlässig zu sein, derart, dass nur für das Spurenelement die erste Elektrode nach einem Hindurchtreten durch die Barriereschicht erreichbar ist, um das von der Konzentration des Spurenelements abhängige Signal zu erzeugen.

In verschiedenen Ausführungsbeispielen können die elektronische Schaltkreisvorrichtung und die Mehrzahl von Elektroden eine integrierte Einheit bilden.

In verschiedenen Ausführungsbeispielen kann die integrierte Einheit ferner die Selektionsschicht aufweisen.

In verschiedenen Ausführungsbeispielen kann die Mehrzahl von Elektroden Teil einer tragbaren Vorrichtung sein.

In verschiedenen Ausführungsbeispielen kann die tragbare Vorrichtung ein Smartphone, ein Tablet oder ein iPad aufweisen.

In verschiedenen Ausführungsbeispielen kann die tragbare Vorrichtung ferner eine Datenaustauschvorrichtung zum kontaktlosen Datenaustausch aufweisen.

In den Zeichnungen beziehen sich ähnliche Bezugszeichen üblicherweise auf dieselben Teile in allen unterschiedlichen Ansichten, wobei der Übersichtlichkeit wegen teilweise darauf verzichtet wird, sämtliche einander entsprechenden Teile in allen Figuren mit Bezugszeichen zu versehen. Teile derselben oder ähnlicher Art können zur Unterscheidung zusätzlich zu einem gemeinsamen Bezugszeichen mit einer nachgestellten Ziffer oder einem nachgestellten kleinen Buchstaben versehen sein. Die Zeichnungen sollen nicht notwendigerweise eine maßstabgetreue Wiedergabe darstellen, sondern die Betonung liegt vielmehr auf einem Veranschaulichen der Prinzipien der Erfindung. In der folgenden Beschreibung werden verschiedene Ausführungsformen der Erfindung mit Bezug auf die folgenden Zeichnungen beschrieben, in denen:
FIG. 1A und FIG. 1B jeweils eine Vorrichtung zum Ermitteln eines Hautzustands gemäß verschiedenen Ausführungsbeispielen zeigen und ein Verwenden der Vorrichtung veranschaulichen;
FIG. 2A und FIG. 2B jeweils eine Vorrichtung zum Ermitteln eines Hautzustands gemäß verschiedenen Ausführungsbeispielen zeigen und ein Verwenden der Vorrichtung veranschaulichen;
FIG. 3A und FIG. 3B jeweils eine Vorrichtung zum Ermitteln eines Hautzustands gemäß verschiedenen Ausführungsbeispielen zeigen;
FIG. 4 Hautregionen zum Anwenden eines Verfahrens und einer Vorrichtung zum Ermitteln eines Hautzustands gemäß verschiedenen Ausführungsbeispielen veranschaulicht;
FIG. 5 ein Flussdiagramm zum Veranschaulichen eines Verfahrens zum Ermitteln einer Hautbehandlungsempfehlung gemäß verschiedenen Ausführungsbeispielen zeigt;
FIG. 6 ein Flussdiagramm eines Verfahrens zum Ermitteln eines Hautzustands gemäß verschiedenen Ausführungsbeispielen zeigt; und
FIG. 7 ein Flussdiagramm eines Verfahrens zum Ermitteln einer kosmetischen Hautbehandlungsempfehlung gemäß verschiedenen Ausführungsbeispielen zeigt.

Die folgende ausführliche Beschreibung bezieht sich auf die begleitenden Zeichnungen, die als Beispiel durch Veranschaulichung bestimmte Details und Ausführungen zeigen, in denen die Erfindung in die Praxis umgesetzt werden kann.

Das Wort "beispielhaft" wird hierin in der Bedeutung von "als ein Beispiel, ein Exemplar oder eine Veranschaulichung dienend" verwendet. Alle hierin als "beispielhaft" beschriebenen Ausführungsformen oder Ausgestaltungen sind nicht notwendigerweise als bevorzugt oder vorteilhaft anderen Ausführungsformen oder Ausgestaltungen gegenüber zu deuten.

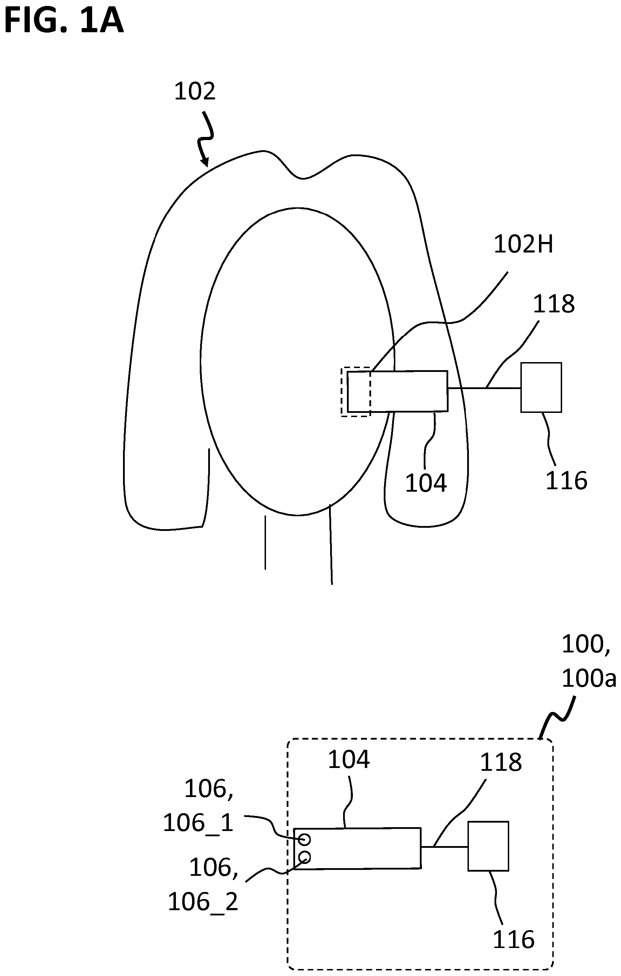

FIG. 1A, FIG. 1B, FIG. 2A, FIG. 2B, FIG. 3A und FIG. 3B zeigen jeweils eine Vorrichtung 100 (100a, 100b, 100c, 100d, 100e oder 100f) zum Ermitteln eines Hautzustands eines Nutzers 102 gemäß verschiedenen Ausführungsbeispielen.

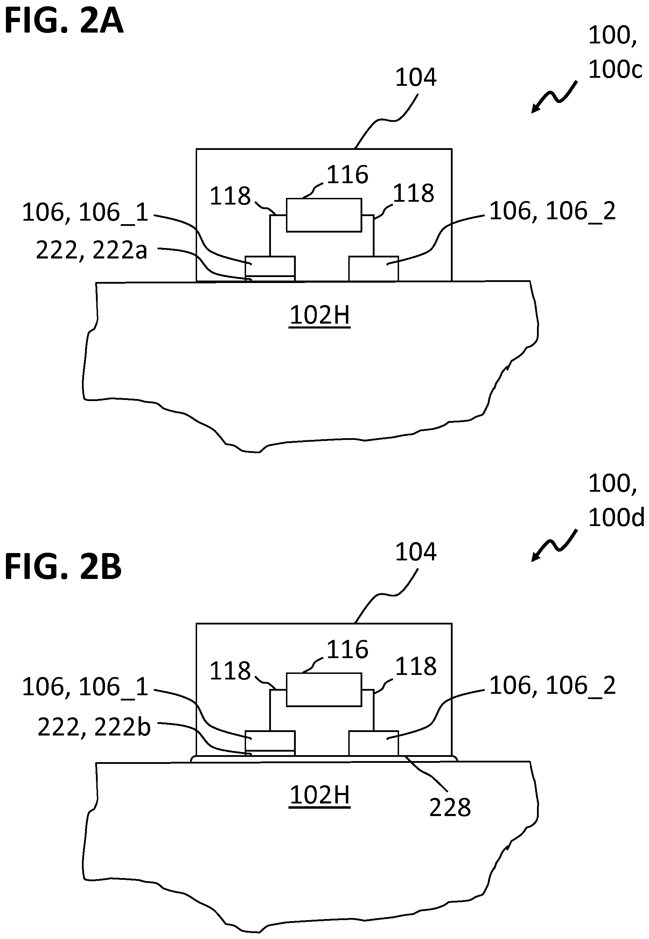

In verschiedenen Ausführungsbeispielen kann die Vorrichtung 100 zum Ermitteln eines Hautzustands eine Mehrzahl von Elektroden 106 aufweisen, wobei die Mehrzahl von Elektroden mindestens eine erste Elektrode 106_1 und eine zweite Elektrode 106_2 aufweisen kann. In verschiedenen Ausführungsbeispielen kann die Vorrichtung 100 ferner eine Selektionsschicht 222 aufweisen, wobei die Selektionsschicht 222 derart selektiv für ein (z.B. ein einziges) Spurenelement gestaltet sein kann, dass nur das (z.B. das einzige) Spurenelement geeignet ist, bei einem Kontakt mit der Selektionsschicht 222 ein von einer Konzentration des Spurenelements abhängiges, zwischen der ersten und der zweiten Elektrode 106_1 bzw. 106_2 messbares Signal zu erzeugen, wobei die erste Elektrode 106_1, die zweite Elektrode 106_2 und die Selektionsschicht 222 derart gestaltet sein können, dass die Mehrzahl von Elektroden 106 und die Selektionsschicht 222 derart über einem Hautbereich 102H eines Nutzers 102 anordenbar sind, dass die Selektionsschicht 222 den Hautbereich 102H kontaktiert, die erste Elektrode 106_1 die Selektionsschicht 222 kontaktiert, und die zweite Elektrode 106_2 den Hautbereich 102H kontaktiert. Anders ausgedrückt kann, wie insbesondere in FIG. 2A und FIG. 2B dargestellt ist, die erste Elektrode 106_1 den Hautbereich 102H des Nutzers 102 indirekt mittels der Selektionsschicht 222 kontaktieren, wohingegen die zweite Elektrode 106_2 den Hautbereich des Nutzers 102 direkt kontaktiert (siehe FIG. 2A), oder die erste Elektrode 106_1 kann den Hautbereich 102H des Nutzers 102 indirekt mittels der Selektionsschicht 222 und eines Lösungsmittels (z.B. einer Lösungsmittelschicht) 228 kontaktieren, wohingegen die zweite Elektrode 106_2 den Hautbereich des Nutzers 102 indirekt mittels des Lösungsmittels (z.B. einer Lösungsmittelschicht) 228 kontaktieren kann (siehe FIG. 2B).

Das Lösungsmittel kann in verschiedenen Ausführungsbeispielen Wasser aufweisen, oder ein anderes geeignetes Lösungsmittel, in welchem sich Spurenelemente aus der Haut des Nutzers 102 zumindest teilweise lösen können und welches geeignet sein kann, mit der Selektionsschicht 222 und den Elektroden 106 derart zusammenzuwirken, dass ein von der Konzentration des Spurenelements in der Haut abhängiges Signal zwischen den Elektroden 106 erzeugt wird.

In verschiedenen Ausführungsbeispielen kann das zwischen den Elektroden 106 erzeugte Signal als Spannung, Widerstand oder Strom gemessen werden, beispielsweise auf im Wesentlichen bekannte Weise.

In verschiedenen Ausführungsbeispielen kann die Selektionsschicht 222a (siehe FIG. 2A) mindestens einen reaktiven Bestandteil aufweisen, der eingerichtet sein kann, unter Freisetzung von Elektronen oder Ionen mit dem zu untersuchenden Spurenelement zu reagieren, wobei die freigesetzten Elektronen oder Ionen eingerichtet sein können, die erste Elektrode 106_1 zu erreichen, um das von der Konzentration des zu untersuchenden Spurenelements abhängige Signal zu erzeugen.

Anders ausgedrückt kann in verschiedenen Ausführungsbeispielen das Spurenelement (z.B. als Teil eines Moleküls, als Ion oder als Atom) aus dem Hautbereich 102H des Nutzers direkt (oder indirekt mittels der Lösungsmittelschicht 228, nicht dargestellt) in die Selektionsschicht 222a eindringen und dort mit dem mindestens einen reaktiven Bestandteil (z.B. chemisch) reagieren, z.B. in Form einer Redoxreaktion, und dabei mindestens ein Elektron und/oder mindestens ein Ion freisetzen. Das Elektron oder das Ion kann sich zur ersten Elektrode 106_1 bewegen, wo es in Kombination mit der zweiten Elektrode 106_2 ein Signal erzeugen kann. Bei einer höheren Konzentration des Spurenelements im Hautbereich 102H können mehr Reaktionen mit dem reaktiven Bestandteil ausgelöst und somit mehr Elektronen bzw. Ionen freigesetzt werden, welche zur ersten Elektrode 106_1 wandern können, so dass ein stärkeres Signal zwischen den beiden Elektroden 106_1, 106_2 ausgelöst werden kann, d.h. das erzeugte Signal kann von der Konzentration des Spurenelements im Hautbereich 102H abhängig sein. Eine Beziehung zwischen dem Signal und dem Spurenelementgehalt der Haut und/oder Referenzsignale mit zugeordneten Spurenelementgehaltwerten (z.B. in tabellarischer Form) können in verschiedenen Ausführungsbeispielen vorab, z.B. in Laborversuchen, ermittelt werden.

In verschiedenen Ausführungsbeispielen kann die Selektionsschicht 222 eine teildurchlässige Barriereschicht 222b (siehe FIG. 2B) aufweisen, welche eingerichtet sein kann, nur für das zu untersuchende Spurenelement (z.B. als Teil eines Moleküls, als Ion oder als Atom) durchlässig zu sein, derart, dass nur für das zu untersuchende Spurenelement die erste Elektrode 106_1 nach einem Hindurchtreten durch die Barriereschicht 222b erreichbar ist. Die die erste Elektrode 106_1 erreichenden Spurenelemente können zwischen der ersten Elektrode 106_1 und der zweiten Elektrode 106_2 ein Signal zu erzeugen. Bei einer höheren Konzentration des Spurenelements in der Haut des Nutzers 102 können mehr Spurenelemente durch die teilsdurchlässige Barriereschicht 222b hindurchtreten, so dass das erzeugte Signal von der Konzentration des Spurenelements abhängig sein kann.

In verschiedenen Ausführungsbeispielen kann die Mehrzahl von Elektroden 106 z.B. ein Elektrodenpaar, z.B. aus Arbeitselektrode (der ersten Elektrode 106_1) und Gegenelektrode (der zweiten Elektrode 106_2), aufweisen, oder z.B. ein Elektrodentriplett, z.B. aus Arbeitselektrode (der ersten Elektrode 106_1), Gegenelektrode (der zweiten Elektrode 106_2) und Referenzelektrode (nicht dargestellt).

In verschiedenen Ausführungsbeispielen kann das Spurenelement eines aufweisen aus einer Gruppe von Spurenelementen, wobei die Gruppe Chrom, Cobalt, Eisen, Kupfer, Mangan, Molybdän, Selen, Silicium, Zink, Zinn, Vanadium, Nickel, Arsen, Barium, Strontium, Aluminium und Kombinationen davon umfasst. Dabei kann die Mehrzahl von Elektroden 106 eingerichtet sein, die Konzentration lediglich eines einzigen der Spurenelemente zu ermitteln. In verschiedenen Ausführungsbeispielen (nicht dargestellt) können zum Ermitteln mehrerer verschiedener Spurenelementgehalte mehrere Mehrzahlen von Elektroden 106 bereitgestellt werden, welche sich zumindest hinsichtlich der Selektionsschicht 222 unterscheiden können.

In verschiedenen Ausführungsbeispielen kann z.B. eine erste Mehrzahl von Elektroden gestaltet sein, dass eine zwischen der ersten Elektrode und der Haut anordenbare Selektionsschicht mit einem ersten Spurenelement reagieren kann oder dafür durchlässig ist, wohingegen eine zweite Mehrzahl von Elektroden so gestaltet sein kann, dass eine zwischen der ersten Elektrode der zweiten Mehrzahl von Elektroden und der Haut anordenbare Selektionsschicht mit einem zweiten Spurenelement reagieren kann oder dafür durchlässig ist.

In verschiedenen Ausführungsbeispielen können die erste Mehrzahl von Elektroden und die zweite Mehrzahl von Elektroden Teil einer einzigen Vorrichtung sein. In verschiedenen Ausführungsbeispielen können die erste Mehrzahl von Elektroden und die zweite Mehrzahl von Elektroden derart in bzw. an der Vorrichtung angeordnet sein, dass die Haut des Nutzers 102 gleichzeitig mit beiden Mehrzahlen von Elektroden kontaktierbar ist. In verschiedenen Ausführungsbeispielen können die erste Mehrzahl von Elektroden und die zweite Mehrzahl von Elektroden derart in bzw. an der Vorrichtung angeordnet sein, dass die Haut des Nutzers 102 sequenziell mit beiden Mehrzahlen von Elektroden kontaktierbar ist.

In verschiedenen Ausführungsbeispielen können mehr als zwei Mehrzahlen von Elektroden in der Vorrichtung bereitgestellt sein, welche eingerichtet sein können zum Detektieren weiterer Spurenelemente.

In verschiedenen Ausführungsbeispielen kann die Vorrichtung 100 zum Ermitteln eines Hautzustands ferner eine elektronische Schaltkreisvorrichtung 116 aufweisen, welche eingerichtet sein kann zum Messen des von der Konzentration des Spurenelements abhängigen Signals, zum Ermitteln des Spurenelementgehalts anhand eines Vergleichs zwischen dem gemessenen Signal und Referenzsignalen für bekannte Spurenelementgehalte von Haut und zum Ermitteln des Hautzustands anhand des Spurenelementgehalts.

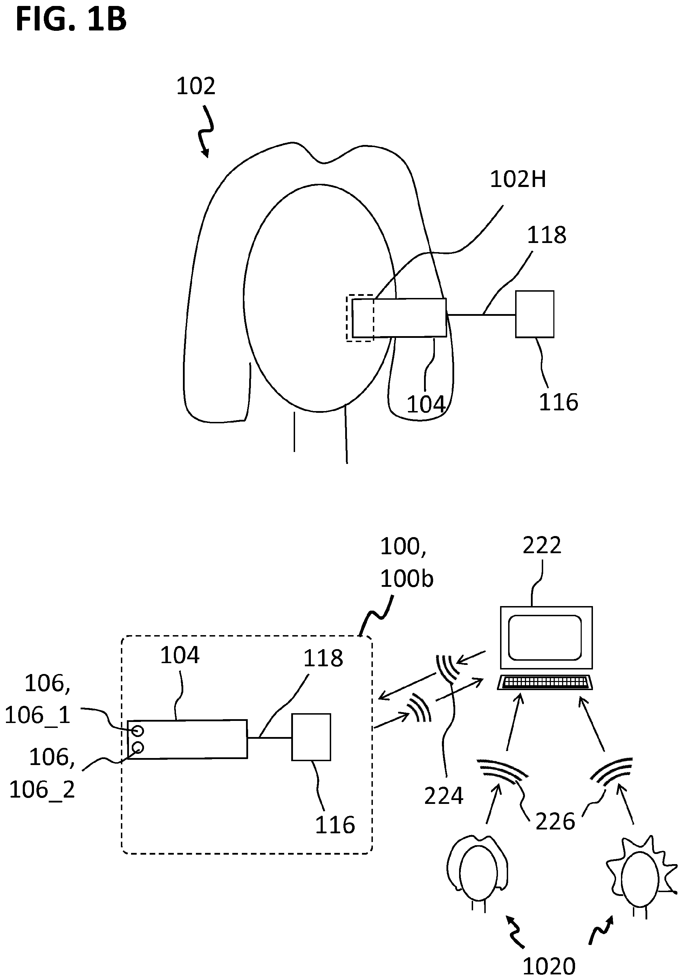

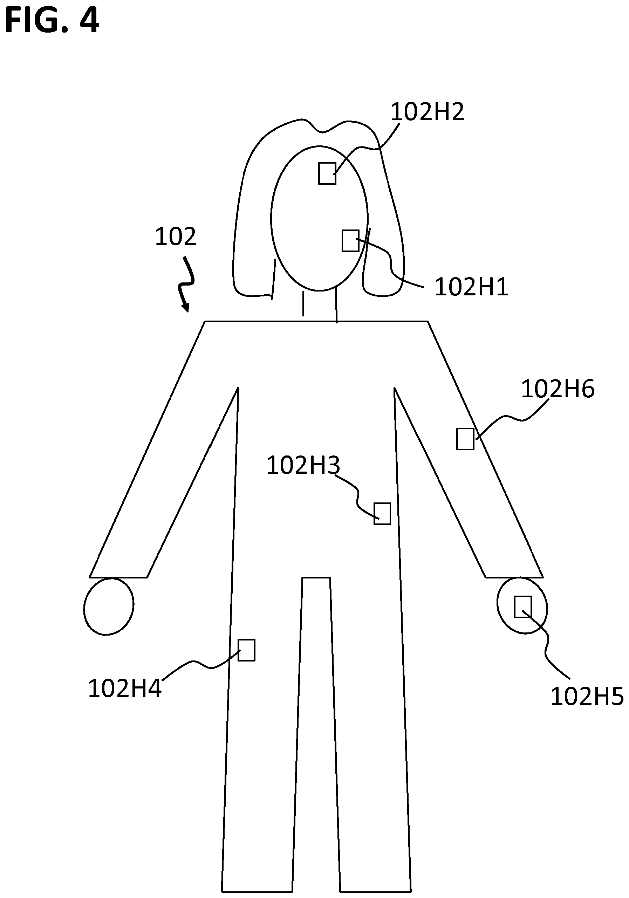

Der Hautzustand kann in verschiedenen Ausführungsbeispielen ermittelt werden, indem für mindestens einen Hautbereich 102H des Nutzers 102 ein Spurenelementgehalt ermittelt wird und anhand dessen der Hautzustand ermittelt wird. Wie in FIG. 1A und FIG. 1B als Hautbereich 102H dargestellt ist, kann in verschiedenen Ausführungsbeispielen der Hautbereich 102H ein Teil einer Gesichtshaut des Nutzers 102 sein. Alternativ oder zusätzlich kann, wie in FIG. 4 dargestellt ist, welche Hautregionen 102H zum Anwenden eines Verfahrens und einer Vorrichtung zum Ermitteln eines Hautzustands gemäß verschiedenen Ausführungsbeispielen zeigt, ein anderer bzw. weiterer Hautbereich 102, beispielsweise an einem Arm (Hautbereich 102H6), einem Bein (Hautbereich 102H4), einem Rumpf (Bereich 102H4) und/oder einer Hand (Bereich 102H5) eines Nutzers zum Ermitteln eines Hautzustands dieses Hautbereichs verwendet werden.

In verschiedenen Ausführungsbeispielen können die Elektroden 106 mit der elektronischen Schaltkreisvorrichtung 116 gekoppelt sein, z.B. mittels einer (kabelgebundenen oder kabellosen) Datenverbindung 118.

Die elektronische Schaltkreisvorrichtung 116 kann in verschiedenen Ausführungsbeispielen eingerichtet sein, wie oben beschrieben mittels Vergleichens des zwischen den Elektroden 106erzeugten Signals einen Spurenelementgehalt der Haut des Nutzers 102 (bei Messung einer Mehrzahl von Hautbereichen 102H den Spurenelementgehalt des jeweiligen Hautbereiches) zu ermitteln, z.B. ein sehr hoher, mäßig hoher, normaler, mäßig niedriger oder sehr niedriger Spurenelementgehalt. In verschiedenen Ausführungsformen kann der Spurenelementgehalt auf andere Weise beschrieben oder quantifiziert werden, z.B. als Spurenelementgehaltszahl (z.B. mit einer beliebigen Einheit) oder ähnliches.

Unter Verwendung des Spurenelementgehalts (bzw. der Mehrzahl von Spurenelementgehalten) kann in verschiedenen Ausführungsbeispielen wie oben beschrieben ein Hautzustand des Nutzers 102 ermittelt werden.

In verschiedenen Ausführungsbeispielen kann bei (bzgl. seines Wertes) im Wesentlichen nur einem ermittelten Spurenelementgehalt oder bei einheitlichen ermittelten Spurenelementgehalten für eine Mehrzahl unterschiedlicher Hautbereiche der Hautzustand dem ermittelten Spurenelementgehalt entsprechen, z.B. kann bei einem ermittelten sehr hohen Spurenelementgehalt für den einzigen, alle oder fast alle Hautbereiche der ermittelte Hautzustand sehr spurenelementreich sein, bei einem ermittelten sehr niedrigen Spurenelementgehalt für den einzigen, alle oder fast alle Hautbereiche der ermittelte Hautzustand sehr spurenelementarm sein, usw.

In verschiedenen Ausführungsbeispielen kann bei (bzgl. ihres Wertes) uneinheitlichen ermittelten Spurenelementgehalten für eine Mehrzahl unterschiedlicher Hautbereiche der Hautzustand als Mischhaut (d.h. eine Haut, die sowohl spurenelementarme als auch spurenelementreiche Bereiche aufweist) ermittelt werden, wobei je nach Spurenelementgehalt der spurenelementarmen bzw. der spurenelementreichen Bereiche unterschiedliche Arten von Mischhaut ermittelt werden können, z.B. wie oben beschrieben.

Die Vorrichtung 100 zum Ermitteln eines Hautzustands gemäß verschiedenen Ausführungsbeispielen kann eine tragbare Vorrichtung 100 sein.

Die elektronische Schaltkreisvorrichtung 116 kann in verschiedenen Ausführungsbeispielen einen Prozessor und einen Speicher aufweisen. Die elektronische Schaltkreisvorrichtung 116 kann in verschiedenen Ausführungsbeispielen mit der Mehrzahl von Elektroden 106 gekoppelt sein, beispielsweise derart, dass ein Erfassen eines Signals, welches zwischen der Mehrzahl von Elektroden 106 erzeugt wird, ermöglicht ist, beispielsweise mittels einer Datenverbindung 118. Die Datenverbindung 118 kann beispielsweise ein Datenkabel, eine interne leitende Verbindung und/oder eine kabellose Übertragung, z.B. mittels WLAN, Thread, ZigBee oder Bluetooth, aufweisen. Die elektronische Schaltkreisvorrichtung 116 kann beispielsweise ein Smartphone, ein iPad, ein Tablet, ein Laptop, ein Smart Mirror oder Ähnliches sein.

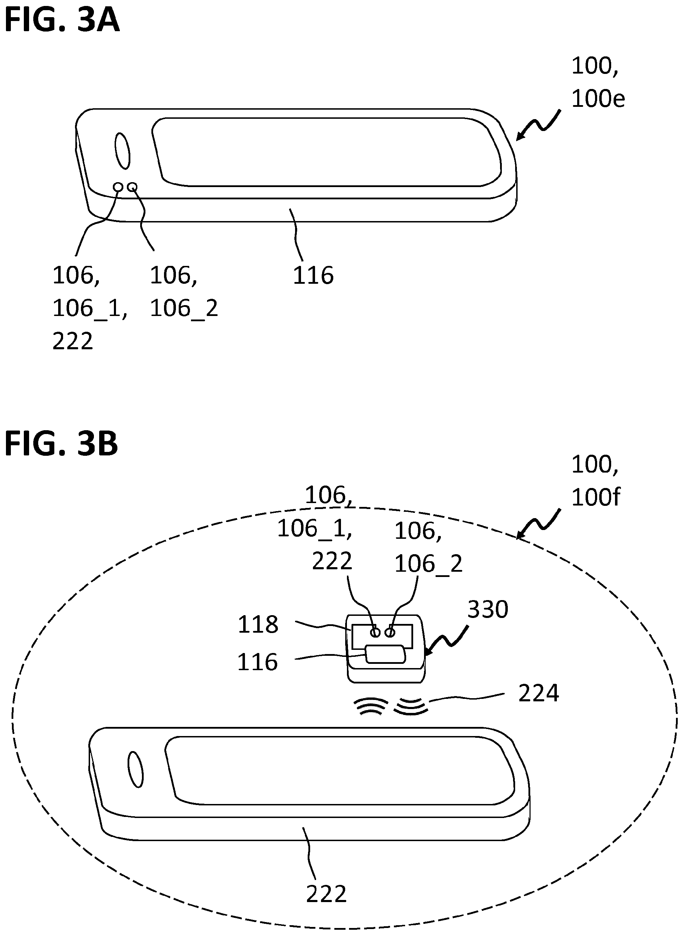

In verschiedenen Ausführungsbeispielen (wie z.B. in FIG. 3A und FIG. 3B dargestellt) können die Mehrzahl von Elektroden 106 und die elektronische Schaltkreisvorrichtung 116 eine tragbare integrierte Einheit bilden.

Beispielsweise können die Mehrzahl von Elektroden 106 und die elektronischen Schaltkreisvorrichtung 116 als eine tragbare integrierte Einheit die Hautuntersuchungsvorrichtung 100 bilden, z.B. wie in FIG. 3A für ein Smartphone mit in ein Gehäuse des Smartphones integrierten Elektroden 106 dargestellt.

In einem anderen Beispiel können die Mehrzahl von Elektroden 106 und die elektronische Schaltkreisvorrichtung 116 eine tragbare integrierte Einheit 330 bilden (siehe FIG. 3B), welche mit einer externen Datenverarbeitungsvorrichtung 222, z.B. einem Smartphone oder ähnlichem, gekoppelt sein kann, z.B. mittels einer kabellosen Datenübertragung 224, z.B. wie hierin an anderer Stelle beschrieben (wobei alternativ eine andere Form der Datenübertragung genutzt werden kann, z.B. eine kabelgebundene). Dabei kann eine Kombination aus der integrierten Einheit 330 und der externen Datenverarbeitungsvorrichtung 222 in verschiedenen Ausführungsbeispielen die Vorrichtung 100f zum Ermitteln des Hautzustands bilden.

In verschiedenen Ausführungsbeispielen kann die integrierte Vorrichtung 100e, 100f eine Größe aufweisen, die es ermöglicht, sie in einer Hand- oder Hosentasche unterzubringen. Beispielsweise kann die integrierte Vorrichtung eine Fläche von weniger als 36 cm² und eine Dicke von weniger als 2 cm aufweisen.

Die elektronische Schaltkreisvorrichtung 116 kann in verschiedenen Ausführungsbeispielen eingerichtet sein, basierend auf dem gemessenen Elektrodensignal einen Spurenelementgehalt zu ermitteln. Ferner kann in verschiedenen Ausführungsbeispielen die elektronische Schaltkreisvorrichtung 116 eingerichtet sein, basierend auf dem mindestens einen Spurenelementgehalt einen Hautzustand zu ermitteln. In verschiedenen Ausführungsbeispielen kann die elektronische Schaltkreisvorrichtung 116 eingerichtet sein, selbst, d.h. direkt, den Spurenelementgehalt der Haut und/oder den Hautzustand zu ermitteln.

Für das Ermitteln des Spurenelementgehalts kann in verschiedenen Ausführungsbeispielen eine Software, beispielsweise eine App, genutzt werden, welche beispielsweise auf einer tragbaren Datenverarbeitungsvorrichtung installiert sein kann, wobei die tragbare Datenverarbeitungsvorrichtung beispielsweise die elektronische Schaltkreisvorrichtung 116 sein kann, wie z.B. in FIG. 3A dargestellt, oder eine zusätzliche, z.B. externe, Datenverarbeitungsvorrichtung 222 sein kann, wie z.B. in FIG. 3B dargestellt.

In verschiedenen Ausführungsbeispielen kann der Spurenelementgehalt bzw. der Hautzustand zeitlich abhängig (z.B. mehrmals täglich, täglich, wöchentlich, oder mit jeder anderen zeitlichen Abhängigkeit) und/oder unter verschiedenen Umgebungsbedingungen (z.B. in einer Umgebung mit hoher oder niedriger Luftfeuchtigkeit, bei Kälte oder Hitze, usw.) ermittelt werden.

In verschiedenen Ausführungsbeispielen können Kalibrationsmessungen (auch als Referenzmessungen bezeichnet), welche beispielsweise in einem Labor durchgeführt werden können, und deren Ergebnisse in der Datenverarbeitungsvorrichtung gespeichert sein können, beispielsweise als Teil der Software/App, genutzt werden, um ein zwischen den Elektroden messbares Signal, z.B. wie oben beschrieben (z.B. eine Spannung, einen Strom oder einen Widerstand) und/oder den mindestens einen ermittelten Spurenelementgehalt einem Hautzustand zuzuordnen.

In verschiedenen Ausführungsbeispielen kann das Ermitteln des Spurenelementgehalts, beispielsweise wie oben beschrieben, direkt mittels der tragbaren elektronischen Schaltkreisvorrichtung 116 ausgeführt werden, z.B. mittels der Software/App, welche auf der tragbaren elektronischen Schaltkreisvorrichtung 116 installiert sein kann.

In verschiedenen Ausführungsbeispielen kann mittels der elektronischen Schaltkreisvorrichtung, z.B. mittels der Software/App, der Spurenelementgehalt bereitgestellt werden. Der Spurenelementgehalt kann beispielsweise als Wert (z.B. mit willkürlichen Einheiten), als verbale Mitteilung, als graphische Darstellung, o.ä. mitgeteilt, z.B. dargestellt werden, z.B. mittels Anzeigens, z.B. an einem Bildschirm der tragbaren elektronischen Schaltkreisvorrichtung 116.

In verschiedenen Ausführungsbeispielen kann das Ermitteln des Spurenelementgehalts, beispielsweise wie oben beschrieben, indirekt mittels der tragbaren elektronischen Schaltkreisvorrichtung 116 erfolgen, beispielsweise indem die elektronische Schaltkreisvorrichtung 116 das Ermitteln des Spurenelementgehalts mittels einer externen Datenverarbeitungsvorrichtung 222 veranlasst. Die elektronische Schaltkreisvorrichtung 116 kann in verschiedenen Ausführungsbeispielen eine relativ einfache elektronische Schaltkreisvorrichtung aufweisen, welche beispielsweise eingerichtet sein kann, im Wesentlichen lediglich das Signal zwischen den Elektroden 106 zu messen (es ggf. mit den Referenzsignalen zu vergleichen) und der externen Datenverarbeitungsvorrichtung 222 zu übertragen. In verschiedenen Ausführungsbeispielen kann die elektronische Schaltkreisvorrichtung 116 eine leistungsfähigere, vielseitige Schaltkreisvorrichtung (z.B. ein Smartphone, ein Tablet oder ein iPad) aufweisen, welches nicht nur eingerichtet sein kann, das Signal zwischen den Elektroden 106 zu messen (und ggf. mit den Referenzsignalen zu vergleichen) und der externen Datenverarbeitungsvorrichtung 222 zu übertragen, sondern zusätzlich eingerichtet sein kann, verschiedene weitere Funktionen auszuführen, z.B. Eingaben vom Nutzer 102 zu erfragen und zu speichern, Ergebnisse darzustellen, ein Foto des Nutzers 102 zum Anzeigen der Ergebnisse bereitzustellen (z.B. mittels einer Kamera) und zu verarbeiten, usw.

In verschiedenen Ausführungsbeispielen kann das Messergebnis des Elektrodensignals und/oder das Vergleichsergebnis von der tragbaren elektronischen Schaltkreisvorrichtung 116, wie in FIG. 1B dargestellt, der externen Datenverarbeitungsvorrichtung 222, beispielsweise einem zentralen Computer, einen Smart Mirror oder einer Cloud, welche/r beispielsweise eine höhere Rechenleistung und/oder eine größere Speicherkapazität als die elektronische Schaltkreisvorrichtung 116 aufweisen kann, bereitgestellt werden, z.B. an diese übertragen werden. Die externe Datenverarbeitungsvorrichtung 222 kann eingerichtet sein, den Spurenelementgehalt aus den bereitgestellten Daten, z.B. dem Signal oder dem Ergebnis des Vergleichs, zu ermitteln, beispielsweise mittels einer Software, z.B. einer App, beispielsweise wie oben beschrieben. In verschiedenen Ausführungsbeispielen kann die elektronische Schaltkreisvorrichtung 116 ferner eingerichtet sein, den Spurenelementgehalt von der externen Datenverarbeitungsvorrichtung 222 zu empfangen und bereitzustellen, beispielsweise wie oben beschrieben. Zum Datenaustausch mit der externen Datenverarbeitungsvorrichtung 222 kann die elektronische Schaltkreisvorrichtung 116 in verschiedenen Ausführungsbeispielen eine Vorrichtung zur kabellosen Datenübertragungsvorrichtung aufweisen, z.B. mittels WLAN, Thread, ZigBee oder Bluetooth. In verschiedenen Ausführungsbeispielen kann die elektronische Schaltkreisvorrichtung 216 eingerichtet sein, die Daten mit der externen Datenverarbeitungsvorrichtung 222 mittels einer Kabelverbindung auszutauschen.

In verschiedenen Ausführungsbeispielen kann die elektronische Schaltkreisvorrichtung 116 eingerichtet sein, basierend auf dem ermittelten Spurenelementgehalt einen Hautzustand zu ermitteln.

In verschiedenen Ausführungsbeispielen kann die elektronische Schaltkreisvorrichtung 116 eingerichtet sein, selbst, d.h. direkt, den Hautzustand zu ermitteln, beispielsweise mittels einer Software, z.B. einer App, die auf der tragbaren elektronischen Schaltkreisvorrichtung 116 installiert sein kann.

In verschiedenen Ausführungsbeispielen kann die elektronische Schaltkreisvorrichtung 116 eingerichtet sein, indirekt den Hautzustand zu ermitteln, beispielsweise indem die elektronische Schaltkreisvorrichtung 116 das Ermitteln des Hautzustands mittels der externen Datenverarbeitungsvorrichtung, z.B. mittels einer Software, z.B. einer App, die auf der externen Datenverarbeitungsvorrichtung 222 installiert sein kann, veranlasst, beispielsweise ähnlich wie oben für das Ermitteln des Spurenelementgehalts beschrieben.

In verschiedenen Ausführungsbeispielen kann die Software/App zum Ermitteln des Spurenelementgehalts der Haut dieselbe sein wie die Software/App zum Ermitteln des Hautzustands. In verschiedenen Ausführungsbeispielen, z.B. in einem Fall, dass der Spurenelementgehalt der Haut mittels der externen Datenverarbeitungsvorrichtung 222 ermittelt wird, wohingegen der Hautzustand von der tragbaren elektronischen Schaltkreisvorrichtung 116 ermittelt wird, oder umgekehrt, kann/können unterschiedliche Software/Apps für das Ermitteln des Spurenelementgehalts und für das Ermitteln des Hautzustands verwendet werden.

In verschiedenen Ausführungsbeispielen kann das Ermitteln des Hautzustands ein Vergleichen des mindestens einen ermittelten Spurenelementgehalts mit Einträgen einer Datenbank aufweisen, welche eine Mehrzahl von Hautzuständen mit jeweils mindestens einem zugeordneten Spurenelementgehalt aufweisen kann. In verschiedenen Ausführungsbeispielen kann bei einer Zuordnung von mehr als einem Spurenelementgehalt zu einem Hautzustand in der Datenbank zusätzlich der Hautbereich registriert sein, für welchen der Spurenelementgehalt in der Datenbank ermittelt wurde. In einem solchen Fall können beim Ermitteln des Hautzustands mittels einer Mehrzahl von Spurenelementgehalten die Hautbereiche berücksichtigt werden, für welche der jeweilige Spurenelementgehalt ermittelt wurde, sofern die Hautbereiche bekannt sind, z.B. vom Nutzer angegeben wurden, oder angenommen werden können, z.B. weil der Nutzer angewiesen wurde, die Spurenelementmessung an bestimmten Hautbereichen (ggf. in einer bestimmten Reihenfolge) vorzunehmen.

Die Datenbank kann in verschiedenen Ausführungsbeispielen in der tragbaren elektronischen Schaltkreisvorrichtung 116 und/oder in der externen Datenverarbeitungsvorrichtung 222 gespeichert sein.

Beim Ermitteln des Hautzustands kann derjenige Hautzustand als der Hautzustand des Nutzers ermittelt werden, dessen Spurenelementgehalt (bzw. Spurenelementgehalte) die geringsten Abweichungen zum/zu den in der Datenbank dem Hautzustand zugeordneten Spurenelementgehalt(en) zeigt.

In verschiedenen Ausführungsbeispielen kann die Vorrichtung 100 eingerichtet sein, dem Nutzer 102 den ermittelten Hautzustand bereitzustellen, z.B. mittels der Software/App. Der Hautzustand kann beispielsweise als Wert (z.B. mit willkürlichen Einheiten), als verbale Mitteilung, als graphische Darstellung, o.ä. mitgeteilt, z.B. dargestellt werden, z.B. mittels Anzeigens, z.B. an einem Bildschirm der tragbaren elektronischen Schaltkreisvorrichtung 116.

Insbesondere in einem Fall, in welchem der Spurenelementgehalt für eine Mehrzahl von Hautbereichen ermittelt wird, kann das Bereitstellen des Hautzustands an den Nutzer eine graphische Darstellung aufweisen, beispielsweise eine Anzeige, z.B. mittels einer Anzeigevorrichtung (z.B. eines Bildschirms/Displays). Dafür kann in verschiedenen Ausführungsbeispielen eine Darstellung eines Körpers oder eines Körperbereichs (z.B. des Gesichts) verwendet werden, beispielsweise als symbolische Darstellung oder als ein Foto des Nutzers 102. Der Darstellung kann eine Veranschaulichung des Spurenelementgehalts bzw. des Hautzustands überlagert sein, z.B. mittels unterschiedlicher Symbole/Markierungen/Schraffuren für Bereiche unterschiedlichen Spurenelementgehalts bzw. Hautzustands.

Beispielsweise kann in einem Fall, in welchem der Spurenelementgehalt für eine Mehrzahl von Hautbereichen ermittelt wird, das Bereitstellen des Hautzustands an den Nutzer eine graphische Darstellung auf einem Smart Mirror aufweisen. Dafür kann in verschiedenen Ausführungsbeispielen eine Echtzeit-Darstellung eines Körpers oder eines Körperbereichs (z.B. des Gesichts) auf/in dem Smart Mirror verwendet werden. Der Darstellung kann eine Veranschaulichung des Spurenelementgehalts bzw. des Hautzustands überlagert sein, z.B. mittels unterschiedlicher Symbole/Markierungen/Schraffuren für Bereiche unterschiedlichen Spurenelementgehalts bzw. Hautzustands.

In verschiedenen Ausführungsbeispielen kann die Vorrichtung 100 ferner eingerichtet sein, basierend auf dem ermittelten Hautzustand eine kosmetischen Hautbehandlungsempfehlung zu ermitteln und dem Nutzer 102 bereitzustellen.

In verschiedenen Ausführungsbeispielen kann/können jedem der Mehrzahl von Hautzuständen mindestens ein geeignetes Kosmetikprodukt und/oder mindestens ein Pflegehinweis zugeordnet sein. Die Zuordnung kann beispielsweise experimentell, z.B. bei Laborversuchen, ermittelt worden sein.

In verschiedenen Ausführungsbeispielen können zum Beurteilen einer Eignung eines Kosmetikprodukts und/oder eines Pflegehinweises zum Pflegen einer Haut mit einem gegebenen Hautzustand Literaturdaten zu Grunde gelegt werden.

In verschiedenen Ausführungsbeispielen kann jedem der Hautzustände ein Qualitätswert zugeordnet sein oder werden. Ein Kosmetikprodukt und/oder ein Pflegehinweis kann für einen Hautzustand als geeignet bewertet werden, wenn zu erwarten ist, z.B. aufgrund von Literaturdaten, Versuchsergebnissen oder Erfahrungswerten, dass bei einer (z.B. regelmäßigen) Anwendung des Kosmetikprodukts und/oder des Pflegehinweises der Hautzustand des Nutzers 102 aufrechterhalten wird oder sich zu einem Hautzustand mit einem höheren Qualitätswert ändert.

In verschiedenen Ausführungsbeispielen, wie in FIG. 1B dargestellt, kann eine Beurteilung einer Eignung eines Kosmetikproduktes, einen Hautzustand zu verbessern, bestätigt oder abgeändert werden mittels eines Aufnehmens von Erfahrungswerten weiterer Nutzer 1020 mit demselben oder einem ähnlichen Hautzustand, beispielsweise von Erfahrungswerten hinsichtlich eines Behandlungserfolgs. Die Erfahrungswerte können von den weiteren Nutzern beispielsweise der externen Datenverarbeitungsvorrichtung 222 bereitgestellt werden, beispielsweise mittels einer kabellosen Datenübertragung 226. Alternativ kann auch eine Übertragung der Daten mittels Kabel genutzt werden. Anhand der Erfahrungswerte kann die Datenbank in der externen Datenverarbeitungsvorrichtung 222 und/oder in der tragbaren elektronischen Schaltkreisvorrichtung 116 aktualisiert werden. Damit kann ermöglicht sein, dass der Nutzer 102 immer eine optimale Empfehlung erhält.

In verschiedenen Ausführungsbeispielen können beim Ermitteln der Produkt- und/oder Pflegeempfehlungen ferner Informationen hinsichtlich eines generellen Gesundheitszustands, eines Hautzustands, Ernährungsgewohnheiten und weiterer Verhaltensweisen des Nutzers (z.B. tägliche Aufenthaltsdauer im Freien/in der Sonne/im Wasser (z.B. in spurenelementhaltigem Wasser), Rauchgewohnheiten, Ernährungsgewohnheiten, usw.) verwendet werden, z.B. mittels der auf der tragbaren elektronischen Schaltkreisvorrichtung 116 und/oder auf der externen Datenverarbeitungsvorrichtung 222 installierten Software/App. Die Informationen können in verschiedenen Ausführungsbeispielen mittels der tragbaren elektronischen Schaltkreisvorrichtung 116 vom Nutzer 102 erfragt werden, welcher sie in die elektronische Schaltkreisvorrichtung 116 eingeben kann, beispielsweise mittels einer Tastatur, als Sprachnachricht, als Auswahl aus einem von der tragbaren elektronischen Schaltkreisvorrichtung 116 dargestellten Menü, oder ähnliches.

Beispielsweise können, wenn der Nutzer 102 als zusätzliche Information zur Verfügung stellt, dass er viel Zeit im Wasser oder im Freien verbringt, bei der Produkt- und/oder Pflegehinweisempfehlung basierend auf dem Hautzustand des Nutzers 102 beispielsweise diejenigen zugeordneten Kosmetikprodukte dem Nutzer 102 empfohlen werden, die nicht nur für seinen Hautzustand geeignet sind, sondern außerdem z.B. wasserfest und/oder mit einem UV-Filter versehen sind, und/oder bei einer festgestellten Überversorgung der Haut mit Spurenelementen und einer Information, dass der Nutzer 102 häufig Heilbäder aufsucht, eine Pflegeanweisung bereitgestellt werden, die Zahl/Dauer der Aufenthalte in Heilwasser einzuschränken.

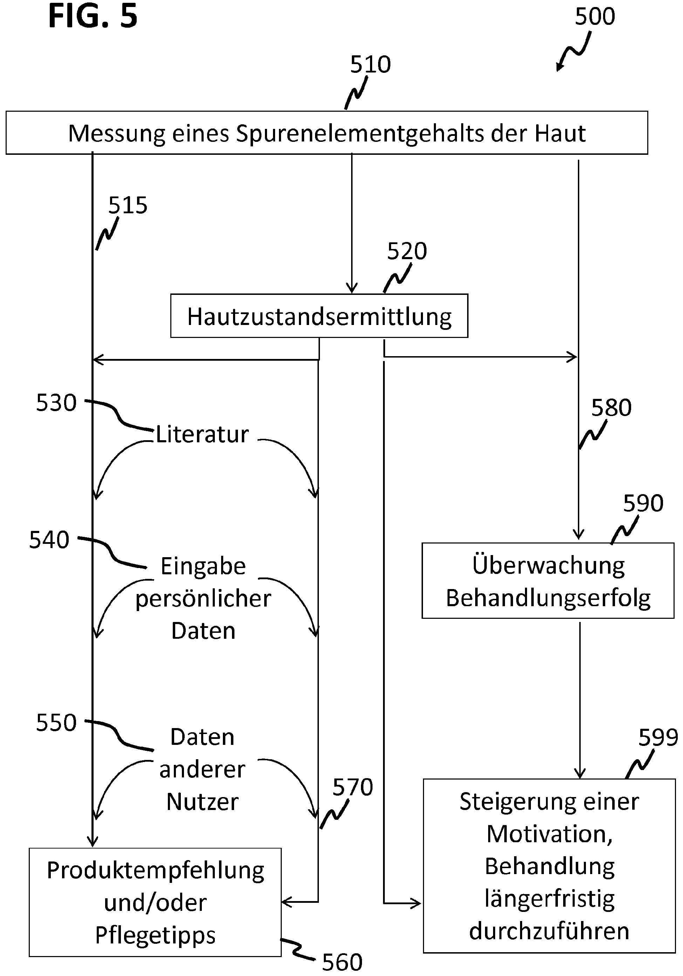

In verschiedenen Ausführungsbeispielen kann die Produktempfehlung und/oder der Pflegehinweis dem Nutzer 102 bereitgestellt werden, beispielsweise mittels der elektronischen Schaltkreisvorrichtung 116, z.B. mittels des Bildschirms der elektronischen Schaltkreisvorrichtung 116. Beispielsweise kann die graphische Darstellung 500 aus FIG. 5 durch (z.B. Text-)Mitteilungen ergänzt werden (nicht dargestellt), z.B. die ermittelten Produktempfehlungen und/oder Pflegehinweise, wie beispielsweise "Trage Creme A im schraffierten Bereich auf" oder ähnliches.

FIG. 5 zeigt ein Diagramm 500 zum Veranschaulichen eines Verfahrens zum Ermitteln eines Kosmetikprodukts zur Hautbehandlung gemäß verschiedenen Ausführungsbeispielen. FIG. 5 kann im Wesentlichen diejenigen Prozesse veranschaulichen, die an anderer Stelle im Zusammenhang mit dem Verfahren zum Ermitteln eines Kosmetikprodukts zur Hautbehandlung gemäß verschiedenen Ausführungsbeispielen beschrieben sind.

Das Verfahren kann gemäß verschiedenen Ausführungsbeispielen aufweisen ein Ermitteln eines Spurenelementgehalts der Haut (bei 510), beispielsweise wie oben beschrieben.

In verschiedenen Ausführungsbeispielen kann das Verfahren ferner ein Ermitteln eines Hautzustands (auch als Hauttyp bezeichnet) aufweisen (bei 520), beispielsweise wie oben beschrieben.

Basierend auf dem ermittelten Hautzustand und/oder auf dem ermittelten Spurenelementgehalt kann eine Produkt- oder Pflegetippempfehlung ermittelt werden (bei 560). Dabei kann in verschiedenen Ausführungsbeispielen (als Pfad 570 gekennzeichnet) nur der anhand des Spurenelementgehalts ermittelte Hautzustand verwendet werden.

In verschiedenen Ausführungsbeispielen (als Pfad 515 gekennzeichnet) können zusätzlich zum Spurenelementgehalt auch Literaturdaten (mit 530 gekennzeichnet), die persönlichen Daten (z.B. wie oben beschrieben, mit 540 gekennzeichnet), und oder Daten anderer Nutzer, z.B. deren Erfahrungswerte (als 550 gekennzeichnet) hinzugezogen werden.

In verschiedenen Ausführungsbeispielen kann ferner, wie bei Pfad 580 dargestellt, mittels eines Ermittelns des Spurenelementgehalts (bei 510) ein Behandlungserfolg überwacht werden (bei 590). Dies kann beispielsweise besonders während und nach einer kosmetischen Behandlung aufgrund der Produktempfehlung (bei 560) sinnvoll sein. Ein mittels des Verfahrens zum Ermitteln des Hautzustands anhand objektiver Werte belegter Behandlungserfolg kann eine Motivation beim Nutzer steigern (bei 599).

In verschiedenen Ausführungsbeispielen können, wie oben beschrieben, anhand des ermittelten Spurenelementgehalts und/oder anhand des ermittelten Hautzustands Produktempfehlungen für individuell für den Nutzer 102 passende Kosmetikprodukte und/oder individuelle Pflegehinweise abgeleitet werden. Die Produktempfehlungen und/oder Pflegehinweise können beispielsweise mittels einer Software, z.B. einer App, bereitgestellt werden. Zusätzlich kann dem Nutzer ein empfohlenes Kosmetikprodukt zum Kauf angeboten werden und der Nutzer kann über eine Eingabe den Kauf in die Wege leiten. Neben dem Kauf von Kosmetikprodukt können dem Nutzer auch weitergehende Informationen zum käuflichen Erwerb angeboten werden. Diese weitergehenden Informationen können sich auf detailliertere individuelle Pflegehinweise beziehen. Die Software/App Programm empfängt beispielsweise die Anfrage, dass der Nutzer das empfohlene Kosmetikprodukt erwerben möchte, speichert die Anfrage und/oder übermittelt die Anfrage an ein Handelsunternehmen, welches die Produkte vertreibt. Der Nutzer wird durch die Software/App aufgefordert, seine persönlichen Daten (Adresse, Bankinformationen, Versendungspräferenz, etc.) einzugeben. Alternativ kann dem Nutzer angegeben werden, wo (zum Beispiel Drogeriemarkt, Kosmetiksalon, Parfümerie, Apotheke, etc.) er das empfohlene Produkt erwerben kann.

In verschiedenen Ausführungsbeispielen kann dem Nutzer zum Einsatz von Kosmetikprodukten, die für den Nutzer individuell hergestellt werden, zugeraten und ein Bestellvorgang, vorzugsweise durch Aufrufen einer Internetseite eines Herstellers von individuellen Kosmetikprodukten, eingeleitet werden.

Immer Kunden wünschen sich ein individuell auf ihre Bedürfnisse abgestimmtes Produkt. Dabei kann es sich um ein speziell für den Kunden hergestelltes Produkt oder ein sogenanntes "mass customized" Produkt handeln. Bei einem "mass customized" Produkt kann durch Variation von wenigen, aus Kundensicht jedoch entscheidenden Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurator abläuft. Dieser Konfigurator hilft dem Kunden bei der Auswahl der Merkmale/Inhaltsstoffe und weist ihn auf die zulässigen/unzulässigen Merkmalskombinationen hin, wobei letztere dann nicht ausgewählt werden können.

Bei Produkten für die Haut umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten Hautzustand ungeeigneter Inhaltsstoffe vermieden werden. Umgekehrt kann die Auswahl für den ermittelten Hautzustand geeigneter Inhaltsstoffe durch den Produktkonfigurator vorgegeben oder vorgeschlagen werden.

In verschiedenen Ausführungsbeispielen kann der Besuch eines Hautarztes oder eines Kosmetikers empfohlen werden. In verschiedenen Ausführungsbeispielen kann direkt über die Software/App, welche den Spurenelementgehalt der Haut und/oder den Hautzustand und/oder die Produktempfehlung und/oder die Pflegehinweise ermittelt und/oder einen , ein Buchungsvorgang eingeleitet werden. Dazu können beispielsweise in der Software/App die Kontaktdaten von Hautärzten und/oder Kosmetikern hinterlegt sein und diese dem Nutzer angezeigt werden. Zusätzlich kann über Filter, wie beispielsweise die Postleitzahl, die Auswahl eingeschränkt werden. In verschiedenen Ausführungsbeispielen kann direkt über die Software/App eine Terminbuchung vorgenommen werden. Alternativ kann die Buchung eines Hautarzttermins und/oder eines Kosmetiktermins über eine separate Software/App, wie beispielsweise Treatwell, erfolgen.

In verschiedenen Ausführungsbeispielen kann die Software/App, welche den Spurenelementgehalt der Haut und/oder den Hautzustand ermittelt, dieselbe sein, die die Produktempfehlung und/oder die Pflegehinweise ermittelt und/oder den Bestellvorgang eines empfohlenen Produkts einleitet. In verschiedenen Ausführungsbeispielen können unterschiedliche Softwareprogramme/Apps verwendet werden für einen Teil der verschiedenen Vorgänge oder alle verschiedenen Vorgänge (Ermitteln des Spurenelementgehalts, Ermitteln des Hautzustands, Ermitteln einer Produktempfehlung, Ermitteln eines Pflegehinweises, Einleiten eines Bestellvorgangs).

In verschiedenen Ausführungsbeispielen kann ein Behandlungserfolg bei einer kosmetischen Behandlung, welche ein positives Beeinflussen des ermittelten Spurenelementgehalts der Haut bzw. des ermittelten Hautzustands zum Ziel haben kann, überwacht werden. In verschiedenen Ausführungsbeispielen kann die Software/App eine Kontrolle und/oder Nachverfolgung der Ergebnisse mittels einer Darstellung (z.B. einer graphischen Darstellung) der Messergebnisse im Verlauf der Zeit ermöglichen.

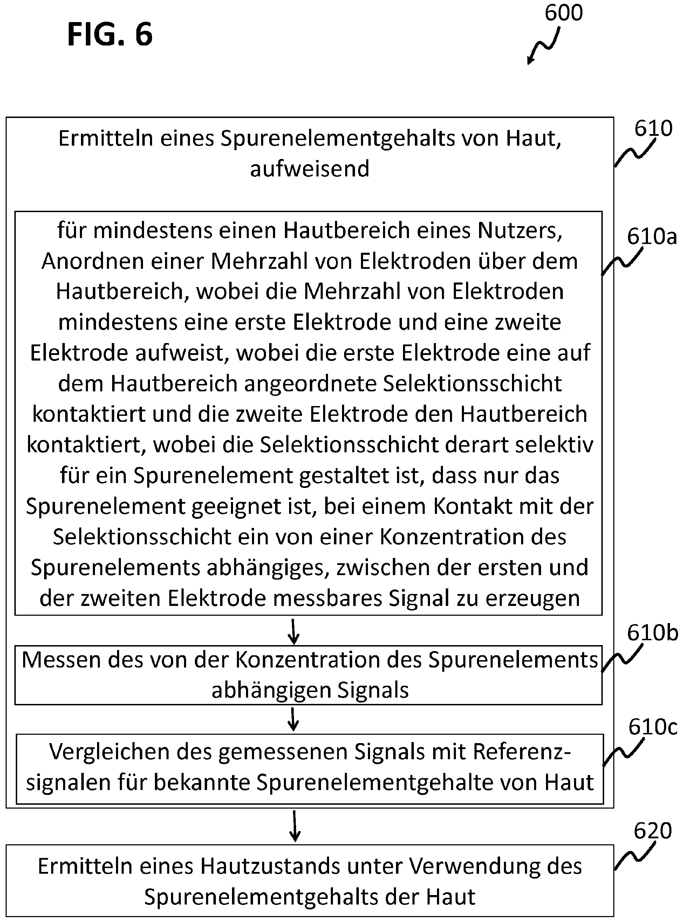

FIG. 6 zeigt ein Flussdiagramm 600 eines Verfahrens zum Ermitteln eines Hautzustands gemäß verschiedenen Ausführungsbeispielen.

In verschiedenen Ausführungsbeispielen wird ein Verfahren zum Ermitteln eines Hautzustands bereitgestellt. Das Verfahren kann aufweisen ein Ermitteln eines Spurenelementgehalts (bei 610), aufweisend: für mindestens einen Hautbereich eines Nutzers, Anordnen einer Mehrzahl von Elektroden über dem Hautbereich, wobei die Mehrzahl von Elektroden mindestens eine erste Elektrode und eine zweite Elektrode aufweist, wobei die erste Elektrode eine auf dem Hautbereich angeordnete Selektionsschicht kontaktiert und die zweite Elektrode den Hautbereich kontaktiert, wobei die Selektionsschicht derart selektiv für ein Spurenelement gestaltet ist, dass nur das Spurenelement geeignet ist, bei einem Kontakt mit der Selektionsschicht ein von einer Konzentration des Spurenelements abhängiges, zwischen der ersten und der zweiten Elektrode messbares Signal zu erzeugen (bei 610a), Messen des von der Konzentration des Spurenelements abhängigen Signals (bei 610b) und Vergleichen des gemessenen Signals mit Referenz-signalen für bekannte Spurenelementgehalte von Haut (bei 610c), und Ermitteln eines Hautzustands unter Verwendung des Spurenelementgehalts der Haut (bei 620).

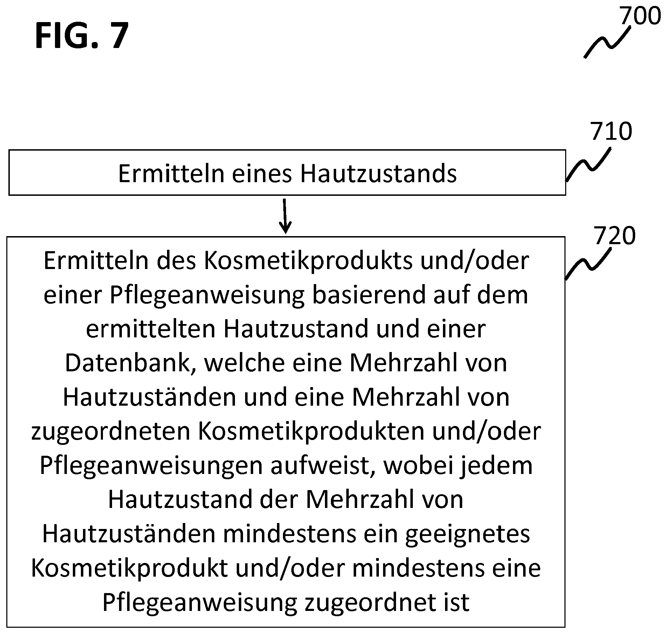

FIG. 7 zeigt ein Flussdiagramm 700 eines Verfahrens zum Ermitteln einer kosmetischen Hautbehandlungsempfehlung gemäß verschiedenen Ausführungsbeispielen.

In verschiedenen Ausführungsbeispielen wird ein Verfahren zum Ermitteln einer kosmetischen Hautbehandlungsempfehlung bereitgestellt. Das Verfahren kann aufweisen: ein Ermitteln eines Hautzustands gemäß verschiedenen Ausführungsbeispielen (bei 710) und ein Ermitteln des Kosmetikprodukts und/oder der Pflegeanweisung basierend auf dem ermittelten Hautzustand und einer Datenbank, welche eine Mehrzahl von Hautzuständen und eine Mehrzahl von zugeordneten Kosmetikprodukten bzw. Pflegeanweisungen aufweist, wobei jedem Hautzustand der Mehrzahl von Hautzuständen mindestens ein geeignetes Kosmetikprodukt und/oder mindestens eine geeignete Pflegeanweisung zugeordnet sein kann (bei 720).

Manche der Ausführungsbeispiele sind im Zusammenhang mit Vorrichtungen beschrieben, und manche der Ausführungsbeispiele sind im Zusammenhang mit Verfahren beschrieben. Weitere vorteilhafte Ausgestaltungen des Verfahrens ergeben sich aus der Beschreibung der Vorrichtung und umgekehrt.

## Patentansprüche

1. Verfahren zum Ermitteln einer kosmetischen Hautbehandlungsempfehlung, aufweisend:
Ermitteln eines Hautzustands, aufweisend:
Ermitteln eines Spurenelementgehalts von Haut, aufweisend:
für mindestens einen Hautbereich eines Nutzers, Anordnen einer Mehrzahl von Elektroden über dem Hautbereich, wobei die Mehrzahl von Elektroden mindestens eine erste Elektrode und eine zweite Elektrode aufweist, wobei die erste Elektrode eine auf dem Hautbereich angeordnete Selektionsschicht kontaktiert und die zweite Elektrode den Hautbereich kontaktiert, wobei die Selektionsschicht derart selektiv für ein Spurenelement gestaltet ist, dass nur das Spurenelement geeignet ist, bei einem Kontakt mit der Selektionsschicht ein von einer Konzentration des Spurenelements abhängiges, zwischen der ersten und der zweiten Elektrode messbares Signal zu erzeugen;
Messen des von der Konzentration des Spurenelements abhängigen Signals;
Vergleichen des gemessenen Signals mit Referenzsignalen für bekannte Spurenelementgehalte von Haut; und
Ermitteln des Hautzustands basierend auf dem ermittelten Spurenelementgehalt der Haut, Ermitteln eines Kosmetikprodukts und/oder einer Pflegeanweisung basierend auf dem ermittelten Hautzustand und einer Datenbank, welche eine Mehrzahl von Hautzuständen und eine Mehrzahl von zugeordneten Kosmetikprodukten und/oder Pflegeanweisungen aufweist, wobei jedem Hautzustand der Mehrzahl von Hautzuständen mindestens ein geeignetes Kosmetikprodukt und/oder mindestens eine Pflegeanweisung zugeordnet ist, wobei der ermittelte Hautzustand ein Mangelzustand hinsichtlich des Spurenelements ist;
und wobei das Kosmetikprodukt das Spurenelement aufweist, sofern ein Kosmetikprodukt ermittelt wird.

2. Verfahren gemäß Anspruch 1,
wobei die Selektionsschicht mindestens einen reaktiven Bestandteil aufweist, der eingerichtet ist, unter Freisetzung von Elektronen oder Ionen mit dem Spurenelement zu reagieren, und
wobei die freigesetzten Elektronen oder Ionen eingerichtet sind, die erste Elektrode zu erreichen, um das von der Konzentration des Spurenelements abhängige Signal zu erzeugen.

3. Verfahren gemäß Anspruch 1,
wobei die Selektionsschicht eine teildurchlässige Barriereschicht aufweist, welche eingerichtet ist, nur für das Spurenelement durchlässig zu sein, derart, dass nur für das Spurenelement die erste Elektrode nach einem Hindurchtreten durch die Barriereschicht erreichbar ist, um das von der Konzentration des Spurenelements abhängige Signal zu erzeugen.

4. Verfahren gemäß einem der Ansprüche 1 bis 3, ferner aufweisend:
vor dem Anordnen der Mehrzahl von Elektroden über dem mindestens einen Hautbereich, Benetzen des mindestens einen Hautbereichs mittels eines Lösungsmittels, welches vorzugsweise Wasser enthält.

5. Verfahren gemäß einem der Ansprüche 1 bis 4,
wobei das Spurenelement eines aufweist aus einer Gruppe von Spurenelementen, wobei die Gruppe aufweist:
Chrom;
Cobalt;
Eisen;
Kupfer;
Mangan;
Molybdän;
Selen;
Silicium;
Zink;
Zinn;
Vanadium;
Nickel;
Arsen;
Barium;
Strontium;
Aluminium und
Kombinationen davon.

6. Verfahren gemäß einem der Ansprüche 1 bis 5,
wobei der mindestens eine Hautbereich eine Mehrzahl von Hautbereichen des Nutzers aufweist.

7. Verfahren gemäß einem der Ansprüche 1 bis 6,
wobei die Mehrzahl von Elektroden Teil einer tragbaren Vorrichtung ist.

8. Verfahren gemäß Anspruch 7,
wobei die tragbare Vorrichtung ein Smartphone, ein Tablet oder ein iPad aufweist oder an einem Smartphone, einem Tablet oder einem iPad anbringbar ist.

9. Verfahren gemäß einem der Ansprüche 1 bis 8,
wobei das Vergleichen des gemessenen Signals mit Referenzsignalen für bekannte Spurenelementgehalte von Haut und das Ermitteln des Hautzustands basierend auf dem ermittelten Spurenelementgehalt der Haut mittels einer App erfolgt.

10. Verfahren gemäß einem der Ansprüche 1 bis 9,
wobei jedem Hautzustand der Mehrzahl von Hautzuständen ein Qualitätswert zugeordnet ist, und das Kosmetikprodukt und/oder die Pflegeanweisung geeignet ist für den Hautzustand der Mehrzahl von Hautzuständen, wenn basierend auf gespeicherten Erfahrungswerten mit dem Kosmetikprodukt eine Verbesserung oder Aufrechterhaltung des Qualitätswerts zu erwarten ist.

11. Verfahren gemäß Anspruch 10, ferner aufweisend:
Aktualisieren der Datenbank basierend auf neuen Erfahrungswerten von einer Mehrzahl von Nutzern.

12. Verfahren gemäß einem der Ansprüche 1 bis 11,
wobei das Ermitteln des Gehalts eines Spurenelements eines Hautbereichs und/oder das Ermitteln des Hautzustands ein Übertragen des Signals, eines Ergebnisses des Vergleichs und/oder des ermittelten Gehalts des Spurenelements des Hautbereichs an eine externe Datenverarbeitungsvorrichtung und ein Empfangen des Gehalts des Spurenelements des Hautbereichs und/oder des Hautzustands aufweist.

## Claims

1. A method for determining a cosmetic skin treatment recommendation, comprising:
determining a skin condition, comprising:
determining a trace element content of skin, comprising:
for at least one skin area of a user, arranging a plurality of electrodes over the skin area, wherein the plurality of electrodes comprises at least a first electrode and a second electrode, wherein the first electrode contacts a selection layer arranged on the skin area and the second electrode contacts the skin area, wherein the selection layer is designed to be selective for a trace element such that only the trace element is capable of generate a signal dependent on the concentration of the trace element and measurable between the first and second electrodes upon contact with the selection layer;
measuring the signal dependent on the concentration of the trace element;
comparing the measured signal with reference signals for known trace element contents of skin; and
determining the skin condition based on the determined trace element content of the skin, determining a cosmetic product and/or care instruction based on the determined skin condition and a database which has a plurality of skin conditions and a plurality of associated cosmetic products and/or care instructions, wherein at least one suitable cosmetic product and/or at least one care instruction is assigned to each skin condition of the plurality of skin conditions, wherein the determined skin condition is a deficiency condition with respect to the trace element; and wherein the cosmetic product comprises the trace element.

2. Method according to claim 1,
wherein the selection layer comprises at least one reactive component that is arranged to react with the trace element by releasing electrons or ions, and
wherein the released electrons or ions are arranged to reach the first electrode to generate the signal dependent on the concentration of the trace element.

3. Method according to claim 1,
wherein the selection layer comprises a partially permeable barrier layer which is arranged to be permeable only to the trace element, such that only the trace element can reach the first electrode after passing through the barrier layer to generate the signal dependent on the concentration of the trace element.

4. Method according to one of claims 1 to 3, further comprising:
before arranging the plurality of electrodes over the at least one skin area, wetting the at least one skin area with a solvent, which preferably contains water.

5. Method according to one of claims 1 to 4,
wherein the trace element comprises one from a group of trace elements, wherein the group comprises:
chromium ;
cobalt;
iron;
copper;
mangane se;
Molybden um;
Selenium;
Silicon;
Zinc;
Tin;
Vanadium;
Nickel;
Arsenic;
Barium;
Strontium;
aluminium and
combinations thereof.

6. Method according to one of claims 1 to 5,
wherein the at least one skin area comprises a plurality of skin areas of the user.

7. Method according to one of claims 1 to 6,
wherein the plurality of electrodes is part of a portable device.

8. Method according to claim 7,
wherein the portable device comprises a smartphone, a tablet or an iPad, or is attachable to a smartphone, a tablet or an iPad.

9. Method according to any one of claims 1 to 8,
wherein the comparison of the measured signal with reference signals for known trace element contents of skin and the determination of the skin condition based on the determined trace element content of the skin is performed by means of an app.

10. Method according to one of claims 1 to 9,
wherein each skin condition of the plurality of skin conditions is assigned a quality value, and the cosmetic product and/or care instruction is suitable for the skin condition of the plurality of skin conditions if, based on stored empirical values with the cosmetic product, an improvement or maintenance of the quality value is to be expected.

11. Method according to claim 10, further comprising:
updating the database based on new empirical values from a plurality of users.

12. Method according to one of claims 1 to 11,
wherein determining the trace element content of a skin area and/or determining the skin condition comprises transmitting the signal, a result of the comparison and/or the determined trace element content of the skin area to an external data processing device and receiving the trace element content of the skin area and/or the skin condition.

## Revendications

1. Procédé pour déterminer une recommandation de traitement cosmétique de la peau, comprenant : la détermination d'un état de la peau, comprenant :
la détermination d'une teneur en oligo-éléments de la peau, comprenant :
pour au moins une zone cutanée d'un utilisateur, la disposition d'une pluralité d'électrodes sur la zone cutanée, la pluralité d'électrodes comprenant au moins une première électrode et une deuxième électrode, la première électrode étant en contact avec une couche de sélection disposée sur la zone cutanée et la deuxième électrode étant en contact avec la zone cutanée, la couche de sélection étant conçue de manière sélective pour un oligo-élément, de telle sorte que seul l'oligo-élément est susceptible, , lorsqu'il est en contact avec la couche de sélection, à générer un signal mesurable entre la première et la deuxième électrode, qui dépend de la concentration de l'oligo-élément ;
mesurer le signal dépendant de la concentration de l'oligo-élément ;
comparer du signal mesuré signal avec signaux de référence pour des teneurs en oligo-éléments connues de la peau ; et
détermination de l'état de la peau sur la base de la teneur en oligo-éléments déterminée de la peau, détermination d'un produit cosmétique et/ou d'une instruction de soin sur la base de l'état de la peau déterminé et d'une base de données qui présente une pluralité d'états de la peau et une pluralité de produits cosmétiques et/ou d'instructions de soin associés, dans lequel au moins un produit cosmétique approprié et/ou au moins une instruction de soin est associé à chaque état de peau parmi la pluralité d'états de peau, dans lequel l'état de peau déterminé est un état de carence en oligo-éléments ; et dans lequel le produit cosmétique contient l'oligo-élément.

2. Procédé selon la revendication 1,
dans lequel la couche de sélection comprend au moins un composant réactif qui est conçu pour réagir avec l'oligo-élément en libérant des électrons ou des ions, et
les électrons ou ions libérés étant conçus pour atteindre la première électrode afin de générer le signal dépendant de la concentration de l'oligo-élément.

3. Procédé selon la revendication 1,
dans lequel la couche de sélection comprend une couche barrière partiellement perméable qui est conçue pour n'être perméable qu'à l'oligo-élément, de telle sorte que seul l'oligo-élément puisse atteindre la première électrode après avoir traversé la couche barrière afin de générer le signal dépendant de la concentration de l'oligo-élément.

4. Procédé selon l'une des revendications 1 à 3, comprenant en outre :
avant de disposer la pluralité d'électrodes sur au moins une zone cutanée, l'humidification de la au moins une zone cutanée à l'aide d'un solvant contenant de préférence de l'eau.

5. Procédé selon l'une des revendications 1 à 4,
dans lequel l'oligo-élément est l'un parmi un groupe d'oligo-éléments, le groupe comprenant :
le chrome
du cobalt ;
le fer ;
le cuivre ;
le manganès e ;
Molybdèn e ;
Sélénium ;
Silicium ;
Zinc;
Étain ;
Vanadium ;
Nickel ;
Arsenic ;
Baryum ;
Strontium ;
aluminium et leurs
combinaisons.

6. Procédé selon l'une des revendications 1 à 5,
dans lequel la au moins une zone cutanée comprend une pluralité de zones cutanées de l'utilisateur.

7. Procédé selon l'une des revendications 1 à 6,
la pluralité d'électrodes faisant partie d'un dispositif portable.

8. Procédé selon la revendication 7,
le dispositif portable comprenant un smartphone, une tablette ou un iPad, ou pouvant être fixé à un smartphone, une tablette ou un iPad.

9. Procédé selon l'une des revendications 1 à 8,
la comparaison du signal mesuré avec des signaux de référence pour des teneurs connues en oligo-éléments de la peau et la détermination de l'état de la peau sur la base de la teneur en oligo-éléments de la peau déterminée s'effectuant à l'aide d'une application.

10. Procédé selon l'une des revendications 1 à 9,
dans lequel une valeur de qualité est attribuée à chaque état de peau parmi la pluralité d'états de peau, et le produit cosmétique et/ou les instructions d'entretien sont adaptés à l'état de peau parmi la pluralité d'états de peau si, sur la base des valeurs empiriques enregistrées avec le produit cosmétique, une amélioration ou un maintien de la valeur de qualité est attendu.

11. Procédé selon la revendication 10, comprenant en outre :
la mise à jour de la base de données sur la base de nouvelles valeurs empiriques provenant d'une pluralité d'utilisateurs.

12. Procédé selon l'une des revendications 1 à 11,
dans lequel la détermination de la teneur en oligo-éléments d'une zone cutanée et/ou la détermination de l'état de la peau comprennent la transmission du signal, d'un résultat de la comparaison et/ou de la teneur déterminée en oligo-éléments de la zone cutanée à un dispositif de traitement de données externe et la réception de la teneur en oligo-éléments de la zone cutanée et/ou de l'état de la peau.
